(19) Europäisches Patentamt
European Patent Office
Office européen des brevets

(11) EP 2 880 182 B1

(12) **EUROPEAN PATENT SPECIFICATION**

(45) Date of publication and mention of the grant of the patent:
**26.07.2017 Bulletin 2017/30**

(21) Application number: **13748003.4**

(22) Date of filing: **02.08.2013**

(51) Int Cl.:
***C12Q 1/68*** *(2006.01)* ***C12N 15/10*** *(2006.01)*

(86) International application number:
**PCT/EP2013/066246**

(87) International publication number:
**WO 2014/020137 (06.02.2014 Gazette 2014/06)**

(54) **RECOMBINASE MEDIATED TARGETED DNA ENRICHMENT FOR NEXT GENERATION SEQUENCING**

REKOMBINASEVERMITTELTE, ABGERICHTETE DNA-ANREICHERUNG FÜR SEQUENZIERUNG DER NÄCHSTEN GENERATION

ENRICHISSEMENT D'ADN CIBLÉ MÉDIÉ PAR LA RECOMBINASE POUR LE SÉQUENÇAGE DE PROCHAINE GÉNÉRATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.08.2012 EP 12179098**
**02.08.2012 US 201261678818 P**

(43) Date of publication of application:
**10.06.2015 Bulletin 2015/24**

(73) Proprietor: **Qiagen GmbH**
**40724 Hilden (DE)**

(72) Inventors:
- **WEDLER, Holger**
  **40724 Hilden (DE)**
- **WEDLER, Erika**
  **40724 Hilden (DE)**

(74) Representative: **Roth, Carla et al**
**König-Szynka-Tilmann-von Renesse**
**Patentanwälte Partnerschaft mbB**
**Mönchenwerther Straße 11**
**40545 Düsseldorf (DE)**

(56) References cited:
**WO-A1-00/63365** **WO-A1-87/01730**
**WO-A1-93/05178** **WO-A2-00/56872**
**WO-A2-02/10457** **US-A1- 2003 082 551**
**US-B1- 6 335 164**

- **F. MERTES ET AL: "Targeted enrichment of genomic DNA regions for next-generation sequencing", BRIEFINGS IN FUNCTIONAL GENOMICS, vol. 10, no. 6, 1 November 2011 (2011-11-01), pages 374-386, XP055040598, ISSN: 2041-2649, DOI: 10.1093/bfgp/elr033**
- **ZHUMABAYEVA B ET AL: "RECA-MEDIATED AFFINITY CAPTURE: A METHOD FOR FULL-LENGTH CDNA CLONING", BIOTECHNIQUES, INFORMA HEALTHCARE, US, vol. 27, no. 4, 1 October 1999 (1999-10-01), XP001098953, ISSN: 0736-6205**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 880 182 B1

**SEQUENCE LISTING**

<110> Qiagen GmbH

<120> Recombinase mediated targeted DNA enrichment for next generation sequencing

<130> 54 629 K

<150> EP12179098.4
<151> 2012-08-02

<150> US61/678,818
<151> 2012-08-02

<160> 59

<170> PatentIn version 3.3

<210> 1
<211> 20
<212> DNA
<213> Artificial

<220>
<223> Adapter

<400> 1
gatcggaaga gcacacgtct 20

<210> 2
<211> 33
<212> DNA
<213> Artificial

<220>
<223> Adapter

<400> 2
acactctttc cctacacgac gctcttccga tct 33

<210> 3
<211> 58
<212> DNA
<213> Artificial

<220>
<223> Amplification primer

<400> 3
aatgatacgg cgaccaccga gatctacact ctttccctac acgacgctct tccgatct 58

<210> 4
<211> 34
<212> DNA
<213> Artificial

<220>
<223> Indexing Pre Capture PCR Reverse Primer

<400> 4
gtgactggag ttcagacgtg tgctcttccg atct 34

<210> 5
<211> 43
<212> DNA
<213> Artificial

<220>
<223> Index primer

<400> 5
caagcagaag acggcatacg agattacaag gtgactggag ttc 43

<210> 6
<211> 26
<212> DNA
<213> Artificial

<220>
<223> Invasion probe for gene DDX31

<400> 6
tttgctgaag gtcacagagt gcatcc 26

<210> 7
<211> 26
<212> DNA
<213> Artificial

<220>
<223> Invasion probe for gene DDX31

<400> 7
agcttcgagc ggcagaagta cctgag 26

<210> 8
<211> 26
<212> DNA

<213> Artificial

<220>
<223> Invasion probe for gene DDX31

<400> 8
gcatcaagac tgtcacccct ccaagg 26

<210> 9
<211> 31
<212> DNA
<213> Artificial

<220>
<223> Invasion probe for gene DDX31

<400> 9
caagctttca cagtcaggtt aacaacacac t 31

<210> 10
<211> 28
<212> DNA
<213> Artificial

<220>
<223> Invasion probe for gene DDX31

<400> 10
cctaaggact acttcagcag gacagtgg 28

<210> 11
<211> 26
<212> DNA
<213> Artificial

<220>
<223> Invasion probe for gene DDX31

<400> 11
ccatcgttga agtgctggag cttatg 26

<210> 12
<211> 26
<212> DNA
<213> Artificial

<220>
<223> Invasion probe for gene DDX31

<400> 12
gaggatgcct ttatgtgggg aaggag 26

<210> 13
<211> 27
<212> DNA
<213> Artificial

<220>

<223> Invasion probe for gene DDX31

<400> 13
ttcccaaacg ctgtctttat gaatagc 27

<210> 14
<211> 27
<212> DNA
<213> Artificial

<220>
<223> Invasion probe for gene DDX31

<400> 14
agcgtcgaga atgtgcagaa ggaacag 27

<210> 15
<211> 30
<212> DNA
<213> Artificial

<220>
<223> Invasion probe for gene DDX31

<400> 15
ccccactatt gacttgcttc ccttttatgc 30

<210> 16
<211> 28
<212> DNA
<213> Artificial

<220>
<223> Invasion probe for gene DDX31

<400> 16
ggagccataa tgaagtatct gggggaac 28

<210> 17
<211> 32
<212> DNA
<213> Artificial

<220>
<223> Invasion probe for gene DDX31

<400> 17
gcagtgttgc tgtatgtaat tttgtctatg ag 32

<210> 18
<211> 31
<212> DNA
<213> Artificial

<220>
<223> Invasion probe for gene DDX31

<400> 18

gtcgcttttt acagtgaatg ggccttgtaa g 31

<210> 19
<211> 29
<212> DNA
<213> Artificial

<220>
<223> Invasion probe for gene DDX31

<400> 19
ggttacccta acacaatcac aaggagaag 29

<210> 20
<211> 31
<212> DNA
<213> Artificial

<220>
<223> Invasion probe for gene DDX31

<400> 20
tgacgtgagt catggtttat tcacatttta g 31

<210> 21
<211> 27
<212> DNA
<213> Artificial

<220>
<223> Invasion probe for gene DDX31

<400> 21
tgactccttc ttgctcatcc ctacttc 27

<210> 22
<211> 26
<212> DNA
<213> Artificial

<220>
<223> Invasion probe for gene DDX31

<400> 22
cgcccacagc ctgatttctc taaagc 26

<210> 23
<211> 31
<212> DNA
<213> Artificial

<220>
<223> Invasion probe for gene DDX31

<400> 23
atcataatgt ggcctagtaa atcaaggaaa c 31

<210> 24

<211> 29
<212> DNA
<213> Artificial

<220>
<223> Invasion probe for gene DDX31

<400> 24
ttctactggc gtggccctgt ttgatttac 29

<210> 25
<211> 33
<212> DNA
<213> Artificial

<220>
<223> Invasion probe for gene DDX31

<400> 25
cttcaactgg tataaagaaa aacctctcca ctg 33

<210> 26
<211> 31
<212> DNA
<213> Artificial

<220>
<223> Invasion probe for gene DDX31

<400> 26
gtgagaccag aaatagaaag tgaggtgact g 31

<210> 27
<211> 31
<212> DNA
<213> Artificial

<220>
<223> Invasion probe for gene DDX31

<400> 27
agacttcacc tgatttacag acccaggact c 31

<210> 28
<211> 28
<212> DNA
<213> Artificial

<220>
<223> Invasion probe for gene DDX31

<400> 28
tctagctttt gttggtgctc tcatagcc 28

<210> 29
<211> 31
<212> DNA
<213> Artificial

<220>
<223> Invasion probe for gene DDX31

<400> 29
gctacatcag gaggtcagtg gggtgcttgt g 31

<210> 30
<211> 30
<212> DNA
<213> Artificial

<220>
<223> Invasion probe for gene DDX31

<400> 30
ccgagaaagt aagatgagac cagtttgtgg 30

<210> 31
<211> 30
<212> DNA
<213> Artificial

<220>
<223> Invasion probe for gene DDX31

<400> 31
cagacttctt tacattccta ccgtcacacc 30

<210> 32
<211> 32
<212> DNA
<213> Artificial

<220>
<223> Invasion probe for gene DDX31

<400> 32
agggtaaact gtaaccacta aggagaaaac tg 32

<210> 33
<211> 17
<212> DNA
<213> Artificial

<220>
<223> Stabilization probe

<400> 33
cactctgtga ccttcag 17

<210> 34
<211> 17
<212> DNA
<213> Artificial

<220>
<223> Stabilization probe

<400> 34
gtacttctgc cgctcga 17

<210> 35
<211> 17
<212> DNA
<213> Artificial

<220>
<223> Stabilization probe

<400> 35
ggaggggtga cagtctt 17

<210> 36
<211> 17
<212> DNA
<213> Artificial

<220>
<223> Stabilization probe

<400> 36
tgttaacctg actgtga 17

<210> 37
<211> 17
<212> DNA
<213> Artificial

<220>
<223> Stabilization probe

<400> 37
tcctgctgaa gtagtcc 17

<210> 38
<211> 17
<212> DNA
<213> Artificial

<220>
<223> Stabilization probe

<400> 38
agctccagca cttcaac 17

<210> 39
<211> 17
<212> DNA
<213> Artificial

<220>
<223> Stabilization probe

<400> 39
tccccacata aaggcat 17

<210> 40
<211> 17
<212> DNA
<213> Artificial

<220>
<223> Stabilization probe

<400> 40
cataaagaca gcgtttg 17

<210> 41
<211> 17
<212> DNA
<213> Artificial

<220>
<223> Stabilization probe

<400> 41
cttctgcaca ttctcga 17

<210> 42
<211> 17
<212> DNA
<213> Artificial

<220>
<223> Stabilization probe

<400> 42
gggaagcaag tcaatag 17

<210> 43
<211> 17
<212> DNA
<213> Artificial

<220>
<223> Stabilization probe

<400> 43
ccagatactt cattatg 17

<210> 44
<211> 17
<212> DNA
<213> Artificial

<220>
<223> Stabilization probe

<400> 44
acaaaattac atacagc 17

<210> 45
<211> 17
<212> DNA

<213> Artificial

<220>
<223> Stabilization probe

<400> 45
cccattcact gtaaaaa 17

<210> 46
<211> 17
<212> DNA
<213> Artificial

<220>
<223> Stabilization probe

<400> 46
ttgtgattgt gttaggg 17

<210> 47
<211> 17
<212> DNA
<213> Artificial

<220>
<223> Stabilization probe

<400> 47
tgtgaataaa ccatgac 17

<210> 48
<211> 17
<212> DNA
<213> Artificial

<220>
<223> Stabilization probe

<400> 48
gggatgagca agaagga 17

<210> 49
<211> 17
<212> DNA
<213> Artificial

<220>
<223> Stabilization probe

<400> 49
agagaaatca ggctgtg 17

<210> 50
<211> 17
<212> DNA
<213> Artificial

<220>

<223> Stabilization probe

<400> 50
tgatttacta ggccaca 17

<210> 51
<211> 17
<212> DNA
<213> Artificial

<220>
<223> Stabilization probe

<400> 51
aaacagggcc acgccag 17

<210> 52
<211> 17
<212> DNA
<213> Artificial

<220>
<223> Stabilization probe

<400> 52
aggtttttct ttatacc 17

<210> 53
<211> 17
<212> DNA
<213> Artificial

<220>
<223> Stabilization probe

<400> 53
tcactttcta tttctgg 17

<210> 54
<211> 17
<212> DNA
<213> Artificial

<220>
<223> Stabilization probe

<400> 54
ggtctgtaaa tcaggtg 17

<210> 55
<211> 17
<212> DNA
<213> Artificial

<220>
<223> Stabilization probe

<400> 55

gagagcacca acaaaag 17

<210> 56
<211> 17
<212> DNA
<213> Artificial

<220>
<223> Stabilization probe

<400> 56
ccccactgac ctcctga 17

<210> 57
<211> 17
<212> DNA
<213> Artificial

<220>
<223> Stabilization probe

<400> 57
actggtctca tcttact 17

<210> 58
<211> 17
<212> DNA
<213> Artificial

<220>
<223> Stabilization probe

<400> 58
cggtaggaat gtaaaga 17

<210> 59
<211> 17
<212> DNA
<213> Artificial

<220>
<223> Stabilization probe

<400> 59
ctccttagtg gttacag 17

## Description

### FIELD OF THE INVENTION

The present invention relates to a method for a targeted enrichment of nucleic acids, e.g. genomic DNA regions, for next generation sequencing.

### BACKGROUND OF THE INVENTION

Over the last years, there has been a fundamental shift away from the use of the Sanger method for DNA sequencing to so-called "next generation sequencing" (NGS) technologies. Here, different NGS technologies and methods exist such as pyrosequencing, sequencing by synthesis or sequencing by ligation. However, all NGS platforms share a common technological feature namely the massively parallel sequencing of clonally amplified or single DNA molecules that are spatially separated in a flow cell or by generation of an oil-water emulsion. NGS allows thousands or even millions to billions of sequencing reactions to be performed simultaneously. In NGS, sequencing is performed by repeated cycles of polymerase-mediated nucleotide extensions or, in one format, by iterative cycles of oligonucleotide ligation. As a massively parallel process, NGS generates hundreds of megabases to gigabases of nucleotide-sequence output in a single instrument run, depending on the platform. The inexpensive production of large volumes of sequence data is the primary advantage over conventional methods. Therefore, NGS technologies have become a major driving force in genetic research. Several NGS technology platforms have found widespread use and include, for example, the following NGS platforms: Roche/454, Illumina Solexa Genome Analyzer, the Applied Biosystems SOLiD™ system, Ion Torrent™ semiconductor sequence analyzer, PacBio® real-time sequencing and Helicos™ Single Molecule Sequencing (SMS). NGS technologies, NGS platforms and common applications/fields for NGS technologies are e.g. reviewed in Voelkerding et al (Clinical Chemistry 55:4 641-658, 2009) and Metzker (Nature Reviews/ Genetics Volume 11, January 2010, pages 31-46). Besides the feature that sequencing is performed in a massively parallel manner in NGS technologies, NGS technology platforms have in common that they require the preparation of a sequencing library which is suitable for massive parallel sequencing. Examples of such sequencing libraries include fragment libraries, mate-paired libraries or barcoded fragment libraries. Most platforms adhere to a common library preparation procedure with minor modifications before a "run" on the instrument. This procedure includes fragmenting the DNA (e.g. by mechanical shearing, such as sonification, hydro-shearing, ultrasound, nebulization or enzymatic fragmentation) followed by DNA repair and end polishing (blunt end or A overhang) and, finally, often platform-specific adaptor ligation. The preparation and design of such sequencing libraries is also described in Voelkerding, 2009 and Metzker, 2010.

Despite the substantial cost reductions associated with NGS technologies in comparison with the classical Sanger sequencing method, whole genome sequencing is still cost intensive. Many platforms do not yet have the capacity to sequence a complex genome in a single run cost-efficiently. Furthermore, there are many applications, wherein whole genome sequencing is not required. For many tasks it is necessary to sequence targeted regions of one or more samples. Thus, it is often desirable to only sequence a specific subset of a given sequencing library instead of sequencing the complete DNA library to avoid unnecessary costs and labour if the region of interest is limited to a fraction of the genome and/or a larger number of samples needs to be analyzed. E.g. sequencing of genomic subregions and gene sets is being used to identify polymorphisms and mutations in genes implicated in cancer or other diseases and in regions of the human genome that linkage and whole-genome association studies have implicated in the disease. Especially in the latter setting, regions of interest can be hundreds of kb's to several Mb in size. Target enrichment is also required if the sequencing targets of interest represent only a small fraction of the total DNA library, such as for example low-abundance transcripts within a complex cDNA library or the region of interest is confined to a single gene within a genomic DNA library. As part of this, methods have been developed that aim to achieve targeted enrichment of e.g. genomic subregions or other target sequences of interest. As discussed, targeted enrichment can be useful in a number of situations where particular portions of a whole genome need to be analysed. Also efficient sequencing of the complete "exome" (all coding portions of the genes) represents a major current application but also smaller sets of genes or genomic regions, e.g. being implicated in diseases (see above).

For targeted enrichment, the target region of interest (also referred to as ROI) is selected, e.g. the exome or a set of genes that are supposed to be sequenced, and sequencing library fragments corresponding to this target region of interest are enriched from the primary sequencing library, thereby providing a target enriched sequencing library. Thereby, sequencing library fragments that do not correspond to the target region of interest are at least depleted in the target enriched sequencing library and the subsequently performed next generation sequencing will produce sequence information that predominantly lies in the target region of interest, thereby providing the desired sequencing result.

To best use NGS for these "targeted" purposes, several target enrichment protocols have been developed which are performed prior to next generation sequencing. Several enrichment steps are usually performed in order to provide a target enriched sequencing library. Targeted enrichment techniques can be characterized via a range of technical considerations related to their performance and ease of use, but the practical importance of any one parameter may vary depending on the methodological approach applied and the scientific question being asked. The most important features of a method, which in turn reflects the biggest challenge in targeted enrichment, include the time for obtaining the target enriched sequence library, the overall cost per target base of useful sequence data, the enrichment factor, ratio of sequence reads on/off target region (specificity), coverage (read depth), evenness of coverage across the target region, method reproducibility and the required amount of input DNA. Target enrichment techniques are for example reviewed in Mertes et al., Briefings in Functional Genomics, 2011 ("Targeted enrichment of genomic DNA regions for next-generation sequencing") and Voelkerding, 2009. Generally, the common target enrichment methods presently used in the prior art are based on "hybrid capture", "selective circularization" or "PCR amplification". Targeted enrichment methods are also disclosed in US2010/0029498, Shen et al. (Proc. Natl. Acad. Sci. U.S.A. (2011) 108:6549-6554) and Albert et al. (Nature Methods 4, 903-905 (2007)).

Enrichment in the prior at technologies is primarily based on in vitro hybridization of single stranded nucleic acid probes or primers to denatured target nucleic acids present in the primary sequencing library. The used primers and probes are designed to be able to hybridize to sequences comprised in the target region of interest. Enrichment thus depends on physical and chemical properties of the nucleic acids such as molecular weight of the nucleic acid molecules, their GC-content, secondary structures, melting and annealing temperatures, and the concentration of nucleic acids as well as the salt concentration. Poor evenness across regions with differing percentages of GC bases is a problem which may translate into low coverage of promoter regions and the first exon of genes as these regions are often GC rich. All these factors have an impact on the re-association kinetics and the specificity of the hybridization reaction. Furthermore, hybridization based technologies usually require high temperatures close to the melting temperature of the used probes as well as long incubation times for high specificity. Standard incubation times for hybridization range from 10 hours (HaloPlex) to 72 hours (NimbleGen SeqCap EZ). The long incubation times are a significant drawback as this increases the time for obtaining the target enriched library that is ready for next generation sequencing. Here, depending on the method, up to 5 working days are required from genomic DNA to a target enriched sequencing library. Furthermore, the need for high hybridization temperatures are associated with the problem of evaporation which in turn may change important parameters in the hybridization composition such as the salt concentration. Evaporation particularly constitutes a problem when working with small volumes. Furthermore, hybridization based prior art methods must first render the nucleic acids comprised in the sequencing library single-stranded which also increases the risk of cross-hybridizations. PCR based prior art target enrichment methods usually require many temperature cycles for denaturation, annealing and extension. For running several hundreds or thousands singleplex PCRs, special equipment for automation or compartimentation (e.g. Fluidigm or Raindance technologies) is required. Alternatively, multiplexing of PCR in one reaction is applied, but frequently causes problems in terms of non-specific byproducts and amplification bias. Furthermore, PCR based enrichment does not scale easily to enable the targeting of very large genome subregions or many DNA samples. PCR may also be expensive. The specificity of enrichment by isothermal amplification like MDA is usually reached by combination of hybridization and/or PCR and other selection steps (e.g. ligation and nuclease treatment) with the isothermal polymerase reaction. Hybrid capture and thus hybridization based technologies are often used for medium to large target regions (10 to 60Mb) while PCR based methods typically only target small regions within the kilo base pairs and low mega base pair range.

There is a strong need for further and improved methods for targeted enrichment for next generation sequencing, which allows to enrich sequencing library fragments corresponding to a target region of interest from a primary sequencing library.

It is thus an objective of the present invention to provide a method for targeted enrichment of nucleic acids for next-generation sequencing which avoids drawbacks of the prior art methods. In particular, it is the object of the present invention to provide a targeted enrichment method for next generation sequencing which is time and cost-efficient and furthermore, can be used for targeted enrichment from different primary sequencing libraries, i.e. which is compatible with various NGS platforms.

## SUMMARY OF THE INVENTION

The present invention describes a novel solution hybridization based method for preparing a target enriched sequencing library from a primary next generation sequencing library. A portion of the double stranded nucleic acid molecules comprised in the sequencing library, the target sequences, comprise a sequence which lies in or corresponds to a target region of interest and these target sequences are supposed to be enriched to provide a target enriched sequencing library. The present method uses invasion probes which are designed to be complementary to a sequence or sequences of the target region of interest and accordingly, are complementary to target sequences comprised in the sequencing library. The present method is based on strand invasion of the double-stranded target sequences comprised in the sequencing library using recombinase coated invasion probes, whereby synaptic complexes, also named D-loops, are formed between the invasion probes and the target sequence. Complex formation is mediated by the recombinase which scans the double stranded nucleic acid molecules comprised in the sequencing library for homologous sequences resulting in branch migration and formation of the synaptic complex if a homologous target sequence is found. The enrichment method according to the present invention is thus based on the speed and accuracy of a recombinase such as RecA instead of relying on chemical or physical parameters for hybridization. The synaptic complexes, which comprise the target sequences and thus comprise sequencing library fragments corresponding at least partially to the target region of interest can subsequently be separated and thus isolated from non-target sequences present in the sequencing library, thereby enriching the target sequences and providing a target enriched sequencing library. The method is quick, simple to carry out and does not require hybridization at high temperatures or long incubation times. Therefore, an improved targeted enrichment method for next generation sequencing is provided with the present invention.

In a first aspect, a method is provided for enriching target sequences from a sequencing library to provide a target enriched sequencing library, wherein the sequencing library is suitable for massive parallel sequencing and comprises a plurality of double-stranded nucleic acid molecules, wherein the method comprises:

a) providing nucleoprotein filaments comprising

(i) a single stranded invasion probe, wherein the invasion probe has a region of substantial complementarity to one strand of a double-stranded target sequence,
(ii) a recombinase;

b) forming a complex between the invasion probe and a complementary portion of the target sequence wherein complex formation is mediated by the recombinase;

c) separating the complexes from the remaining sequencing library, thereby enriching the target sequences.

In a second aspect, a method is provided for sequencing a target region of interest, comprising:

a) providing a sequencing library suitable for massive parallel sequencing and comprising a plurality of double stranded nucleic acid molecules, wherein a portion of the double stranded nucleic acid molecules comprised in the sequencing library, the target sequences, comprise a sequence which lies in the target region of interest;

b) enriching target sequences corresponding to the target region of interest according to the method of the first aspect of the present invention, thereby providing a target enriched sequencing library;

c) sequencing the enriched target sequences in parallel.

The method according to the second aspect pertains to the actual next generation sequencing method, wherein the sequences present in a target enriched sequencing library (obtained using the method according to the first aspect of the present invention) are sequenced in massively parallel manner.

In a third aspect, the present invention pertains to the use of the method according to the second aspect for exome sequencing, exon sequencing, targeted genomic resequencing, gene panel orientated targeted genomic resequencing, transcriptome sequencing and/or molecular diagnostics.

According to a fourth aspect, a kit for performing a method according to first aspect of the present invention is provided, which comprises

a) adaptors for creating a sequencing library suitable for massive parallel sequencing;

b) optionally one or more ligation reagents for coupling the adaptors to a nucleic acid fragment;

c) a recombinase, preferably a RecA like recombinase;

d) a non-hydrolyzable co-factor for the recombinase, preferably adenosine 5'-(gamma-thio)triphosphate;

e) a plurality of different invasion probes wherein the invasion probes differ in their region of complementarity to a target region of interest;

f) a plurality of different stabilization probes being at least partially complementary to the plurality of invasion probes; and

g) a solid support suitable for capturing synaptic complexes formed between the invasion probes and target sequences.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only. Various changes and modifications within the spirit and scope of the disclosed invention will become readily apparent to those skilled in the art from reading the following.

## DETAILED DESCRIPTION OF THE INVENTION

In a first aspect, a method is provided for enriching target sequences from a sequencing library to provide a target enriched sequencing library, wherein the sequencing library is suitable for massive parallel sequencing and comprises a plurality of double-stranded nucleic acid molecules, wherein the method comprises:

a) providing nucleoprotein filaments comprising

(i) a single stranded invasion probe, wherein the invasion probe has a region of substantial complementarity to one strand of a double-stranded target sequence,
(ii) a recombinase;

b) forming a complex between the invasion probe and a complementary portion of the target sequence wherein complex formation is mediated by the recombinase;

c) separating the complexes from the remaining sequencing library, thereby enriching the target sequences.

The present invention provides an alternative method for targeted enrichment for next generation sequencing. In contrast to the prior art targeted enrichment methods which are based on classical hybridisation that relies on chemical or physical parameters, the method according to the present invention uses a recombinase such as RecA in order to identify and thus enrich target sequences from a sequencing library comprising a plurality of double-stranded nucleic acid molecules for next generation sequencing. The targeted enrichment method of the invention has important advantages over the prior art. Using a recombinase to identify target sequences in the sequencing library has the effect that the required incubation time for hybridization may be very short (e.g. less than 15min) and in particular is significantly shorter than prior art targeted enrichment methods which rely on classical hybridization (which usually require incubation times for hybridization of 10h to 72h). Thus, the present method is significantly faster than prior art target enrichment methods. Furthermore, moderate reaction temperatures can be used and the activity of the recombinase is less affected by GC rich regions. The method is accurate, not prone to handling errors and furthermore, is compatible with existing NGS platforms. Therefore, the targeted enrichment method of the present invention provides a significant improvement of the prior art.

RecA mediated affinity capture methods were developed in the late 80ies and were used in order to isolate target sequences e.g. from cDNA or cloning libraries (see e.g. WO87/01730; Zhumabayeva, Biotechniques 27: 834-845 1999, Sena et al, nature genetics volume 3, 1993 pages 365-372 and WO98/08975). RecA mediated DNA targeting was so far used for isolating a target sequence from a mixture of cDNAs or genomic DNAs for transformation purposes. Even though the principle of RecA mediated DNA targeting was known for over 25 years, it was not used yet for providing target enriched sequencing libraries for next generation sequencing. At present, much more time-consuming and laborious target enrichment methods are used (see above). The present invention which combines recombinase mediated DNA targeting with next generation sequencing provides a targeted enrichment method which is simpler, universally applicable, less time consuming, more cost efficient and less prone to handling errors than prior art methods.

The individual steps and preferred embodiments of the target enrichment method according to the present invention will be described in the following:

### *Sequencing library*

The sequencing library comprises a plurality of double-stranded nucleic acid molecules and is suitable for massive parallel sequencing and accordingly, is suitable for next generation sequencing. The plurality of double stranded nucleic acid molecules present in the sequencing library may be linear or circular, preferably, the nucleic acid molecules comprised in the sequencing library are linear.

A sequencing library which is suitable for next generation sequencing can be prepared using methods known in the prior art. Usually, methods for preparing a sequencing library suitable for next generation sequencing includes fragmenting the DNA followed by DNA repair and end polishing and, finally, often NGS platform-specific adaptor ligation.

For example, the DNA, such a genomic DNA or cDNA or any DNA derived therefrom, can be fragmented for example by shearing, such as sonification, hydro-shearing, ultrasound, nebulization or enzymatic fragmentation, in order to provide DNA fragments that are suitable for subsequent sequencing. The length of the fragments can be chosen based on the sequencing capacity of the next generation sequencing platform that is subsequently used for sequencing. Usually, the obtained fragments have a length of 1500bp or less, 1000bp or less, 750bp or less, 600bp or less and preferably 500bp or less as this corresponds to the sequencing capacity of most current next generation sequencing platforms. Preferably, the obtained fragments have a length that lies in a range of 20 to 550bp, 50 to 500bp, preferably 100 to 400bp, more preferred 150 to 350bp. Respective fragment sizes are particularly suitable for genomic DNA, also considering that the size of an exon is approx. 150bp to 200bp in length and respective short fragments can be efficiently sequenced using common next generation sequencing platforms. However, also longer fragments can be used, e.g. if using next generation sequencing methods which allow longer sequence reads. However, longer fragments will usually contain a higher proportion of off-target sequences and this effect would be particularly apparent for exons which have a rather short size. Furthermore, off course also smaller fragment sizes (e.g. starting from 15bp) can be feasible depending on the starting material for preparing the sequencing library and the sequences of interest. E.g. if processing cDNA obtained from RNA comprising or consisting of small RNA (having a size of 200nt or less, 100nt or less, 50nt or less or even 25nt or less as is the case for miRNA), the library may comprise respective shorter fragments.

The fragmented DNA can be repaired afterwards and end polished using methods known in the prior art, thereby providing for example blunt ends or overhangs such as A overhangs.

Furthermore, preferably, adapters are ligated at the 5' and/or 3' ends of the DNA fragments, preferably at both ends of the obtained fragments. The specific design of the adapters depends on the next generation sequencing platform to be used and for the purposes of the present invention, basically any adaptors used for preparing sequencing libraries for next generation sequencing can be used. The adapter sequences provide a known sequence composition allowing e.g. subsequent library amplification and/or sequencing primer annealing. As adaptors, double-stranded or partially double-stranded nucleic acids of known sequence can be used. The adapters may have blunt ends, cohesive ends with 3' or 5'overhangs, may be provided by Y shaped adapters or by stem-loop shaped adapters. Y shaped adapters are e.g. described in US7,741,463 and stem-loop shaped adapters are e.g. described in US2009/0298075. Preferably, the adaptors have a length of at least 7, preferably at least 10, preferably at least 15 bases. The adapter length preferably lies in a range of 10 to 100 bases, preferably 15 to 75 bases, more preferred 20 to 60 bases. Either the same or different adaptors can be used at the 3' and 5' end of the fragments. Using the same type of adaptor for both ends, such as e.g. an Y shaped or a stem-looped shaped adapter, has the advantage that no fragments are lost during library preparation due to adapter mispairing which is an advantage when working with low amounts of DNA.

Thus, ppreferably, the sequencing library used in the present invention consists of randomly fragmented double stranded DNA molecules which are ligated at their 3' and 5' end to adapter sequences. The adaptors provide a known sequence and thus provide a known template for amplification and/or sequencing primers. Optionally, the adapters may also provide an individual index thereby allowing the subsequent pooling of two or more target enriched sequencing libraries prior to sequencing. This embodiment will be described in further detail below. The sequencing library may be generated in vitro using enzymatic manipulations, but preferably does not require DNA permitted transformation of living cells and subsequent clonal cell selection, cultivation and DNA isolation. Suitable methods for preparing sequencing libraries are also described in Metzker, 2011, Voelkerding, 2009, and WO12/003374. As described above, depending on the NGS technology used, several thousands, several millions or even up to billions of reads per run can be obtained.

A single NGS run usually produces enough reads to sequence several target enriched sequencing libraries at once. Therefore, pooling strategies and indexing approaches are a practical way to reduce the per sample cost. Respective multiplexing strategies can also be used in conjunction with the teaching of the present invention. Features enabling multiplexing can be incorporated in different stages of the enrichment process, in particular before or after target enrichment. According to one embodiment, the sequencing library is generated by using adaptors containing specific sequence motifs for library labelling and differentiation ("barcoded" or "index" adaptors). Each sequencing library is provided with individual und thus library specific adapters which provide a library specific sequence. Preferably, each adaptor comprises besides the index region a common universal region which provides a known template for PCR primers and/or sequencing primers that can be used on all libraries. After the target enriched sequencing libraries were obtained, they can be pooled and sequenced in a single run. Providing the DNA fragments of the sequencing library with respective index adaptors thus allows subsequently sequencing several target enriched sequencing libraries in the same sequencing run because the sequenced fragments can be distinguished based on the library specific sequence of the index adaptors. After sequencing, the individual sequences belonging to each library can be sorted via the library specific index which is then found in the obtained sequence. Respective index approaches are known in the prior art and index adapters are also commercially available and are for example provided in the TruSeq® DNA sample prep kits which are suitable for use in the Illumina platform.

An important advantage of the method according to the present invention is that it can be used to enrich target sequences, which comprise a sequence which corresponds to a target region of interest, from a sequencing library which comprises low amounts of DNA material. In general, the low amount of nucleic acid material in the library distinguishes sequencing libraries from common plasmid libraries or other cloning libraries which comprise significantly higher amounts of nucleic acids. According to one embodiment, the sequencing library comprises the double-stranded nucleic acid molecules in an overall amount of 3 μg or less, 2 μg or less, 1.5 μg or less, 1 μg or less, 0.75 μg or less, 0.5 μg or less, 0.4 μg or less, 0.3 μg or less, 0.2 μg, 0.1 μg or less or 0.075 μg or less. The method according to the resent invention also allows to enrich target sequences and also low-abundance target sequences even from libraries which comprise only minimal amounts of DNA starting material. This is an important advantage, because in many cases, the sequencing library comprises the DNA in low amounts as nucleic acid material also gets lost during the preparation of the sequencing library. The sequencing library may be prepared using 5 μg or less, 4μg or less, 3 μg or less, 2 μg or less, 1.5 μg or less, 1 μg or less, 0.75 μg or less, 0.5 μg or less, 0.4 μg or less, 0.3 μg or less, 0.2 μg or less or 0.1 μg or less nucleic acid starting material. Here, specific methods exist in the prior art which allow to prepare sequencing libraries from respective low amounts of DNA starting material, such as even 100ng or less. Here, for example, the Nextera technology (Epicentre) provides a transposon based method which allows the preparation of a sequencing library from very low amounts of DNA starting material.

Nucleic acids such as DNA and/or RNA can be isolated from a sample of interest according to methods known in the prior art to provide the starting material for preparing the sequencing library. RNA is usually first transcribed into cDNA prior to preparing the sequencing library. The term "sample" is used herein in a broad sense and is intended to include a variety of sources and compositions that contain nucleic acids. The sample may be a biological sample but the term also includes other, e.g. artificial samples which comprise nucleic acids such as e.g. PCR products or compositions comprising already purified nucleic acids. Exemplary samples include, but are not limited to, whole blood; blood products; red blood cells; white blood cells; buffy coat; swabs; urine; sputum; saliva; semen; lymphatic fluid; amniotic fluid; cerebrospinal fluid; peritoneal effusions; pleural effusions; biopsy samples; fluid from cysts; synovial fluid; vitreous humor; aqueous humor; bursa fluid; eye washes; eye aspirates; plasma; serum; pulmonary lavage; lung aspirates; animal, including human or plant tissues, including but not limited to, liver, spleen, kidney, lung, intestine, brain, heart, muscle, pancreas, cell cultures, as well as lysates, extracts, or materials and fractions obtained from the samples described above or any cells and microorganisms and viruses that may be present on or in a sample and the like. Materials obtained from clinical or forensic settings that contain nucleic acids are also within the intended meaning of the term "sample". Preferably, the sample is a biological sample derived from a human, animal, plant, bacteria or fungi. Preferably, the sample is selected from the group consisting of cells, tissue, tumor cells, bacteria, virus and body fluids such as for example blood, blood products such as buffy coat, plasma and serum, urine, liquor, sputum, stool, CSF and sperm, epithelial swabs, biopsies, bone marrow samples and tissue samples, preferably organ tissue samples such as lung, kidney or liver. The term "sample" also includes processed samples such as preserved, fixed and/or stabilised samples.

The term "nucleic acid" or "nucleic acids" as used herein, in particular refers to a polymer comprising ribonucleosides and/or deoxyribonucleosides that are covalently bonded, typically by phosphodiester linkages between subunits, but in some cases by phosphorothioates, methylphosphonates, and the like. DNA includes, but is not limited to all types of DNA, e.g. genomic DNA, linear DNA, circular DNA, plasmid DNA, cDNA and free circulating DNA, such as e.g. tumor derived or fetal DNA. Preferably, the DNA is genomic DNA or cDNA. According to one embodiment the DNA was amplified from genomic DNA. In certain embodiments, the genomic DNA is amplified by whole genome amplification method such as random primed strand displacement amplification. According to one embodiment the amplified DNA comprises or consists of amplicons obtained from selected genomic DNA regions. According to one embodiment, the DNA is not amplified prior to preparing the primary sequencing library. RNA includes but is not limited to hnRNA, mRNA, noncoding RNA (ncRNA), including but not limited to rRNA, tRNA, IncRNA (long non coding RNA), lincRNA (long intergenic non coding RNA), miRNA (micro RNA), siRNA (small interfering RNA) and also includes free circulating RNA such as e.g. tumor derived RNA. Small RNA or the term small RNA species in particular refers to RNA having a chain length of 300nt or less, 200nt or less, 100nt or less, 50nt or less or 25nt or less and includes but is not limited to miRNA, siRNA, other short interfering nucleic acids, snoRNAs and the like. In case the RNA is a double-stranded molecule, the chain length indicated as "nt" refers to "bp".

Isolated DNA can then be further processed as described above in order to provide the primary sequencing library from which the target sequences are enriched using the method according to the present invention.

NGS has also provided a powerful new approach, termed RNA-Seq which can be used e.g. for mapping and quantifying transcripts and biological samples. In this application, RNA such as total RNA, ribosomal RNA-depleted RNA, or poly-A+ RNA is isolated from the sample and converted to cDNA. A typical protocol would involve the generation of first strand cDNA via random hexamer-primed reverse transcription and subsequent generation of second strand cDNA with RNase H and DNA polymerase. The cDNA can then be fragmented and optionally, but preferably, ligated to NGS adapters. For small RNAs such as micro RNAs (miRNAs) and short interfering RNAs, preferential isolation via an RNA enrichment method which aims at isolating small RNA can be used. Respective isolation methods are known in the prior art. Furthermore, the sequencing library can be prepared from free circulating RNA, which can be isolated e.g. from samples such as blood plasma or urine and which may comprise tumor-derived RNA indicative of a disease. RNA ligase is used to join adapter sequences to the RNA and this step is often followed by a RT-PCR amplification step before preparing the sequencing library. After sequencing, reads are aligned to a reference genome and/or are compared with known transcript sequences or are assembled de novo. Accordingly, also RNA, including small RNA, may form the starting material for preparing the sequencing library. RNA-Seq is capable of single-base resolution, and, compared with arrays, demonstrates a greater ability to distinguish RNA isoforms, determine allelic expressions and reveal sequence variants. Expression levels may be deducted from the total number of reads that map to the exons of the gene, normalized by the length of exons that can be uniquely mapped. Results obtained with this approach have shown close correlation with those of quantitative PCR and RNA spiking experiments.

The target sequences comprised in and to be enriched from the sequencing library comprise a sequence which lies in a target region of interest. The target region of interest basically corresponds to the region which is supposed to be sequenced and accordingly, which is supposed to be covered by the enriched target sequences in order to obtain the sequence information for the target region of interest. If the sequencing library is made of genomic DNA, a target region of interest usually consists of one or more genomic regions, preferably of more than 10, more than 25, more than 50, more than 100 or even more than 1,000 genomic regions, for example exons and/or regulatory genomic regions covering at least 500 bases or up to several giga base pairs (for example a whole exon with up to 70 gigabase pairs) of the genome. However, as discussed above, a target region of interest may also refer to a set of genes or even single genes of interest, for example single genes, set of genes or genomic regions which, e.g. can potentially being implicated in a disease. The present invention can be applied not only to coding exons in a genome, but to any arbitrarily defined sequence portion of a genome or even metagenome. The present invention can also be applied to the transcriptome and to cDNAs derived from the transcriptome. Accordingly, the target region of interest may also correspond to one or more transcripts, miRNAs, tumor derived nucleic acids or any other nucleic acid sequences of interest that are supposed to be sequences. Further examples of suitable target regions of interest will also be described subsequently. The present invention particularly has the advantage that it allows to enrich sequences corresponding to a small target region of interest as well as for mega base pair-sized target regions of interest.

Having described the sequencing library which comprises a plurality of double stranded nucleic acid molecules and from which the target sequences are enriched to provide a target enriched sequencing library using the method according to the present invention, the individual steps of said method and preferred embodiments will be explained in the following.

***Step a)***

In step a), nucleoprotein filaments are provided which comprise

(i) a single stranded invasion probe, wherein the invasion probe has a region of substantial complementarity to one strand of a double-stranded target sequence, and

(ii) a recombinase.

For providing respective nucleoprotein filaments, a single stranded invasion probe is incubated with a recombinase. Each invasion probe is coated with recombinase molecules, thereby providing a nucleoprotein filament. The invasion probes are designed to comprise a region of complementarity, herein also referred to as region of homology, with a target sequence. As described above, a portion of the double stranded nucleic acid molecules comprised in the sequencing library, the target sequences, comprise a sequence which lies in or corresponds to a target region of interest and these target sequences are supposed to be enriched to provide a target enriched sequencing library for subsequent next generation sequencing. The invasion probes are designed to be complementary to a sequence or sequences of the target region of interest and accordingly, are designed to be complementary to target sequences comprised in the sequencing library. As discussed above, the DNA is usually randomly fragmented to provide the double stranded nucleic acid molecules of the sequencing library. Therefore, it is usually not known in advance, where the region of complementarity will be exactly located on or within the target sequence. Depending on the size of the invasion probe and/or the library fragments, the invasion probe may also be complementary to the whole sequence of the target sequence.

Accordingly, the single-stranded invasion probe and the double stranded target sequence have a region of similar or exact base pair sequence which allows the invasion probe to hybridize with the corresponding base pair region in the double-stranded target sequence. Thus, the invasion probe can recognize and complex specifically with the corresponding base pair region in the double-stranded target sequence, a reaction which is mediated by the recombinase. The extent of base pair mismatches between the invasion probe and the complementary region of the target sequence which is allowed by the recombinase without losing homology/complementarity may be as high as 20 to 30%, depending on the overall length of the probe and the distribution and length of mismatched base pairs. In order to ensure a sequence specific homologous pairing between the double-stranded nucleic acid target sequence and the invasion probe (a reaction which is mediated by the recombinase) it is preferred that the invasion probe generally contains a sequence that is at least 90%, preferably at least 95%, more preferred at least 98% or most preferred, 100% identical to a portion or the whole sequence of the target sequence. The invasion probe may be prepared by denaturing a double-stranded nucleic acid probe which is complementary to either one or both strands of the target sequence. The invasion probe may also be chemically synthesized, what is preferred. The invasion probes in accordance with the present invention preferably have a length of at least 15nt, preferably at least 20nt, more preferred at least 25 nt. According to one embodiment, the invasion probes have a length that lies in a range of 15 to 300 nt, 20 to 200nt, 25 to 150nt, 27 to 100nt or 30 to 75nt. Preferably, short invasion probes are used which have a length of 150nt or less, 120nt or less, preferably 100nt or less or 75nt or less. Particularly preferred is a probe length that lies in the range of 20 to 60nt, more preferred 25 to 50nt or 30 to 40nt. Most preferred are invasion probes which have a length of approximately 30 to 35nt. The shorter the invasion probe, the less mismatches are tolerated by the recombinase. This is an advantage, because thereby the specificity can be increased. When using invasion probes having a length of 30 to 35nt, the recombinase usually tolerates one to two mismatches.

The invasion probes are designed such that a good coverage of the target region of interest is ensured. The invasion probes that are used according to the present invention can be designed basically analogously to the probes that are or can be used in the prior art target enrichment methods that are based on conventional probe hybridisation. Options for designing the invasion probes include but are not limited to designing the invasion probes such that they are located adjacent to each other on the target sequence, respectively the target region of interest (either in close proximity (e.g. at a distance of 50nt or less, 35nt or less, 25nt or less, 20nt or less, 15nt or less, 10nt or less or 5nt or less) or separated by at least 50nt, at least 100nt, at least 150nt or at least 200nt) or the invasion probes may be designed overlapping. The invasion probe may comprise only regions of complementarity with the double-stranded target sequence. As discussed above, because the fragmentation process for preparing the sequencing library usually is random, one can usually not predict where the invasion probe will hybridise, e.g. the 3' end, the 5' end or in the middle of the target sequence, respectively a portion thereof. Thus, using several invasion probes which may optionally also overlap ensures that target sequences corresponding to the target region of interest are efficiently captured. It is also within the scope of the present invention to use invasion probes which target the same region of homology on the target sequence but which contain degenerated or mixed bases at one or more positions within the region of homology/complementarity. It is preferred to design and thus use several invasion probes which target a certain target sequence and/or target region of interest. Using several invasion probes has several advantages also depending on the purpose of sequencing. E.g., it is often not known where exactly a mutation or allelic variation is located. By using several invasion probes, it can be ensured that a mutation is securely detected, in particular when considering that recombinases such as RecA usually only tolerate a few mismatches in the sequence. Furthermore, by using two or more invasion probes which target a specific target sequence, the enrichment efficiency can be increased. This is the particular advantage if the sequencing library comprises DNA in low amounts and/or if the target sequences are only comprised in low amounts and accordingly are low abundant targets. Thereby, it is also possible to avoid e.g. an amplification of the enriched target sequences via polymerase chain reaction in order to increase the number of target sequences or at least reduce the need for performing respective amplification technologies.

According to one embodiment, two or more, preferably at least 10, at least 20, at least 30, at least 40, at least 50, at least 60, at least 75, at least 80, at least 90, at least 100, at least 200, at least 300, at least 400, at least 500, at least 750, at least 1000, at least 5000 or at least 10000 different invasion probes are used, wherein the invasion probes differ in their region of complementarity to the target region of interest. Thereby, a good coverage of the target region can be ensured. The sequence composition of the set of invasion probes determines the target sequences that are selected from the sequencing library. The enriched target sequences cover the target region of interest, thereby allowing to subsequently sequence the target region of interest. It was found by the inventors that a large number of invasion probes can be used effectively in parallel in the method of the invention. As compared to earlier RecA based selection methods that use few large invasion probes for capturing cDNA from clone libraries, it was unexpectedly found that a complex mixture of several hundred or even several thousand invasion probes which may even have a length of 100nt or less, 75nt or less or 60nt or less can effectively be used in the method of the invention to specifically provide complexes between the invasion probes and the complementary portions of target sequences and that the respectively formed complexes can be effectively separated and recovered. This makes the method suitable for providing a target enriched sequencing library that is suitable for next generation sequencing. The number of invasion probes to be used to ensure a good enrichment of target sequences corresponding to the target region of interest also depends on the size of the target region of interest. E.g. more invasion probes are necessary to target and thus cover a large target region of interest. Thus, preferably, a set of invasion probes is used wherein the invasion probes differ in their region of complementarity to the target region of interest. Furthermore, two or more different invasion probes can be used wherein the invasion probes differ in their region of complementarity to a specific target sequence. If invasion probes are located close to each other or are even designed overlapping for targeting a target sequence, e.g. a portion (e.g. exon) of the target region of interest (see also above), the evenness of coverage across the target region of interest can be improved. Thus, using more invasion probes having a respective design may improve the obtained results.

According to one embodiment, the invasion probe is labelled with a "capture moiety" which may be used to facilitate the isolation of the complexes in step c). A wide range of modifications of the invasion probe are suitable and known in the prior art that can be used to provide the invasion probe with a capture moiety. This includes labelling the invasion probe with a capture moiety that is characterized by high affinity binding to a binding agent which can preferably be attached to a solid support to facilitate the separation of the captured complexes. The binding agent is capable of recognizing and binding with high affinity to a capture moiety provided on the invasion probe. The capture moiety may be any molecule which can be attached to the invasion probe and does not interfere with the formation of the recombinase coated nucleoprotein filaments and does not interfere with the formation of the synaptic complex. Furthermore, it is preferred that the capture moiety can be recognized and bound by a binding agent provided on a solid support when the invasion probe is bound to the target sequence.

Capture moieties include, but are not limited to haptens such as chemical moieties, epitope tags, binding partners or unique nucleic acid sequences. Basically, any molecule or chemical entity can be used as capture moiety which allows to isolate or separate the complexes from the remaining sequencing library. One general class of ligands that is suitable as capture moiety includes haptens or antigens which are bound with high affinity by binding agents such as antibodies. A preferred capture moiety is biotin which can be readily derivatized to nucleotides, and which binds specifically and with high affinity to avidin or streptavidin and analogs thereof also when the respective binding agents are bound to a solid support. Biotin can be derivatized to probe nucleotides, for example using linkers, without impairing the ability of the invasion probe to hybridize with the double-stranded target DNA in a recombinase mediated reaction. E.g. the probes can be biotinylated in the course of oligonucleotide synthesis e.g. by the phosphoramidite method using commercially available biotin phosphoramidite. Furthermore, tailed oligonucleotides can be synthesized, amplified by PCR and digested with restriction enzymes. The digested fragments are filled in with biotinylated oligonucleotides and the non-biotinylated strand is digested.

Also labelling with an epitope tag and utilizing an antibody or a binding fragment thereof that recognizes that epitope for capture can be used, for example, labelling the oligonucleotides with digoxigenin and using an anti-digoxigenin antibody for capture. Furthermore, haptens may be used for conjugation with nucleotides or oligonucleotides. Commonly used haptens for subsequent capture include biotin (biotin-11-dUTP), dinitrophenyl (dinitrophenyl-11-dUTP). The oligonucleotides can also be labelled for separation using a number of different modifications that are well known to those of skill in the art. These modifications include for example, fluorescent modifications. Commercially, available fluorescent nucleotide analogs that may be incorporated include but are not limited to are Cy3™-dCTP, Cy3™-dUTP, Cy™5-dCTP, fluorescein-12-dUTP, AlexaFluor®594-5-dUTP, AlexaFluor®-546-14-dUTP and the like. Fluorescein labels may also be used as a separation moiety using commercially available anti-fluorescein antibodies. Also suitable is the labelling with radioisotopes, enzyme labels and chemiluminescent labels. Suitable labels for the invasion probe are also described in the prior art on RecA mediated DNA targeting, e.g. WO87/01730 and WO98/08975.

Furthermore, the nucleoprotein filaments comprise a recombinase which mediates the formation of the synaptic complex between the invasion probe and the target sequence. Preferably, a RecA like recombinase is used. RecA-like recombinases utilized in the present invention include recombinases which have catalytic activity similar to RecA protein derived from Escherichia coli. RecA protein can mediate both homologous pairing and/or strand exchange between appropriate DNA molecules in in vitro homologous recombination assays (Kowalczykowski,S., Ann. Rev. Biohpys. Biophysical Chem., 20:539-575 (1991), Radding C., M., Biochem. Biophys. Acta 1008:131-139 (1989), Radding C., M., J. Biol. Chem. 266:5355-5358 (1991); also see Golub, E., et al., Nucleic Acids Res. 20:3121-3125 (1992)). In addition to DNA-DNA hybridization, RecA protein can promote RNA-DNA hybridization. For example, RecA protein coated single stranded DNA can recognize complementarity with naked RNA (Kirkpatrick, S. et al., Nucleic Acids Res. 20:4339-4346 (1992)). Therefore, any recombinase which can promote homologous pairing and/or strand exchange between appropriate DNA molecules or between DNA and RNA molecules may be used in the present invention as RecA like recombinase. RecA-like recombinases have been isolated and purified from many prokaryotes and eukaryotes. The examples of such recombinases include, but are not limited to, the wild type RecA protein derived from Escherichia coli (Shibata T. et al., Method in Enzymology, 100:197 (1983)), and mutant types of the RecA protein (e.g., RecA 803: Madiraju M. et al., Proc. Natl. Acad. Sci. USA, 85: 6592 (1988) such as for example RecA 803, RecA 441; RecA 441 (Kawashima H. et al., Mol. Gen. Genet., 193: 288 (1984), etc.); uvsX protein, a T4 phage-derived analogue of the protein (Yoncsaki T. ct al., Eur. J. Biochem., 148:127 (1985)); RecA protein derived from Bacillus suhilis (Lovett C. M. et al., J. Biol. Chem., 260: 3305 (1985)); Recl protein derived from Ustilago (Kmiec E. B. et al., Cell, 29 :367 (1982)); RecA-like protein derived from heat-resistant bacteria (such as Thermus aquaticus or Thermus thermophilus) (Angov E. et al., J. Bacteriol., 176: 1405 (1994); Kato R. et al., J. Biochem., 114: 926 (1993)); and RecA-like protein derived from yeast, mouse and human (Shinohara A. et al., Nature Genetics, 4: 239 (1993)). Other examples of RecA-like recombinases include uvsX protein, Rad51, Rad51B, Rad51C, Rad51D, Rad51 E, XRCC2 or DMC1. In a preferred embodiment of the present invention the wild type RecA-protein is used as recombinase.

The recombinase binds to a single stranded invasion probe, thereby forming a nucleoprotein filament. Preferably, one recombinase molecule is bound per 3-6 probe nucleotides. The nucleoprotein filaments are formed in the presence of a non-hydrolyzable co-factor for the recombinase which prevents in step b) the strand replacement reaction which would otherwise be catalyzed by the recombinase. Respective non-hydrolyzable co-factors are known in the prior art and include but are not limited to non-hydrolyzable ATP analogs such as ATPyS, which cannot hydrolyze to ADP and phosphate or GTPyS. Methods and conditions for efficiently "coating" the invasion probe with the recombinase can be derived from prior art methods, such as e.g. WO87/01730 and WO98/08975, and are also described in the examples. For example, the single-stranded invasion probe, which is optionally labeled with a capture moiety (see above), can be contacted with the recombinase using a suitable reaction buffer and a non-hydrolyzable cofactor, e.g. ATPyS. The recombinase is preferably added in an amount to allow that one recombinase molecule is bound per 3 to 6 probe nucleotides in order to provide a functionally saturated amount of recombinase. Preferably, the mixture is incubated for at least 5 minutes, preferably at least 10 minutes at elevated temperatures above 35°C and preferably below 45°C to allow the nucleoprotein filaments to be formed.

As discussed above, preferably two or more, preferably at least 20, at least 50, at least 100, at least 250 or at least 500 different invasion probes (see above) are used. When coating a respective set of different invasion probes with the recombinase, a mixture of nucleoprotein filaments is obtained wherein the nucleoprotein filaments comprise different invasion probes. A respective set of invasion probes allows to enrich target sequences that lie within the target region of interest thereby ensuring that a target enriched sequencing library is obtained that allows to sequence the target region of interest.

The obtained nucleoprotein filaments can be stored e.g. at refrigerated temperatures or frozen without appreciable loss of activity. They may also be lyophilized.

***Step b)***

The obtained nucleoprotein filaments are then contacted with the sequencing library in order to allow a hybridization of the invasion probes to their target sequence(s). Hybridization is mediated by the recombinase and thus does not rely on chemical or physical parameters but on the speed and accuracy of the recombinase. The recombinase scans the double-stranded DNA molecules comprised in the sequencing library for homologous sequences and accordingly, scans for target sequences. When the nucleoprotein filament, which comprises the non-hydrolyzable co-factor, comes into contact with a homologous and thus complementary double-stranded target DNA molecule, the filament rapidly and efficiently complexes with the DNA, thereby forming a stable triple-stranded synaptic complex. In said triple-stranded hybrid, the invasion probe is hybridized to the complementary region of the target sequence, thereby providing a double-strand. The third strand of the triple-stranded hybrid corresponds to the target sequence strand which was displaced by the invasion probe. Often, this synaptic complex is also referred to in the prior art as "D-loop". The D-loop is stabilized by the recombinase. As discussed above, using a recombinase such as a RecA like recombinase for hybridizing the invasion probes to the target sequences has several important advantages. First, the sequencing library does not need to be denatured to provide single-stranded molecules. Second, the pairing reaction can be carried out at moderate temperatures. Third, hybridization is achieved within minutes and is less influenced by the sequence of the target nucleic acid, e.g. the GC content. Therefore, targeted enrichment is improved compared to prior art hybridization based enrichment methods that are used to provide target enriched next generation sequencing libraries.

The nucleoprotein filament can be added to the double-stranded target nucleic acid at a molar ratio e.g. ranging from 1:1 to 1000:1, based on mole ratio of homologous-base nucleotides. The molar ratio is calculated on the basis of the double-stranded target DNA, and not the amount of total double-stranded DNA in the sequencing library. Thus, 1000:1 molar ratio of nucleoprotein filament in a fragment mixture of 0.1% target sequence DNA would include approximately the same quantities of single-stranded and double-stranded DNA. Increasing the filament-to-target ratio will increase the rate of synaptic complex formation and, where the invasion probe is a relatively short single-stranded segment (less than 200 base pairs), will increase the stability of the complex. Optionally, additional nucleic acids such as heterologous DNA and/or RNA may be added to the sequencing library either prior to or during complex formation in order to increase the overall amount of nucleic acids. This may improve the recombinase mediated reaction, thereby potentially increasing the sensitivity and/or specificity.

The reaction mixture is incubated preferably at an elevated temperature of 30°C or above, preferably 35°C or above. Preferably, the incubation occurs at a temperature of 60°C or less, 55°C or less, 50°C or less, preferably 45°C or less and more preferred 40°C or less. Particularly suitable is a hybridization temperature of 37°C. Short incubation times of 30min or less, 25min or less, 20min or less, 15min or less and even 10min or less can be used. However, if desired for whatever reason, also longer incubation periods can be used. They are not necessary though what is an important advantage of the present method.

The triple-stranded synaptic complex is unstable in the absence of the recombinase. Thus, according to a preferred embodiment, the synaptic complex is stabilized by adding a single-stranded stabilization probe which hybridizes to the displaced strand of the target sequence, whereby a double-stranded D-loop is formed as complex. Respective stabilized D-Loops and the design of suitable stabilization probes are described in the prior art, for example in WO02/079495 and Sener et al., 1993, and Belozerkowskii, Biochemistry 1999, 38, 10.785 - 10.792. Adding a respective stabilization probe is preferred, as the enrichment results are improved. Preferably, for each added invasion probe, an at least partially complementary stabilization probe is used in order to allow hybridization to the single-stranded displaced strand. Thus, if a set of invasion probes is used, a corresponding set of stabilization probes is preferably used which are at least partially complementary in order to allow stabilization of the displaced strand. According to one embodiment, the complementary stabilization probe is shorter than the corresponding invasion probe. It may have a size that is at least 30%, at least 40% or at least 50% shorter than the invasion probe. Preferably, the stabilization probe has a length of 10nt to 30nt, preferably 15 to 25nt. The stabilization probe can be labeled with a capture moiety in an analogous fashion as was described above for the invasion probe. It is referred to the above disclosure.

The synaptic complexes, which comprise the target sequences and thus comprise sequencing library fragments corresponding at least partially to the target region of interest can subsequently be separated and thus be isolated from non-target sequences present in the sequencing library.

According to one embodiment, the complex formation may be terminated and the recombinase can be removed from the synaptic complexes by performing for example a proteolytic digest using a proteolytic enzyme, such as preferably proteinase K, and optionally additionally using a detergent. A proteolytic enzyme refers to an enzyme that catalyzes the cleavage of peptide bounds, for example in proteins, polypeptides, oligopeptides and peptides. Exemplary proteolytic enzymes include but are not limited to proteinases and proteases in particular subtilisins, subtilases, alkaline serine proteases and the like. Subtilases are a family of serine proteases, i.e. enzymes with a serine residue in the active side. Subtilisins are bacterial serine protease that has broad substrate specificities. Subtilisins are relatively resistant to denaturation by chaotropic agents, such as urea and guanidine hydrochloride and anionic detergents such as sodium dodecyl sulfate (SDS). Exemplary subtilisins include but are not limited to proteinase K, proteinase R, proteinase T, subtilisin, subtilisin A, QIAGEN Protease and the like. Discussions of subtilases, subtilisins, proteinase K and other proteases may be found, among other places in Genov et al., Int. J. Peptide Protein Res. 45: 391-400, 1995. Preferably, the proteolytic enzyme is proteinase K. As detergent, an anionic, cationic, non-ionic or zwitterionic detergent can be used or combinations of the foregoing. Preferably, an anionic detergent is used. The proteolytic digest removes the recombinase from the complex. As described above, if using a stabilization probe, the resulting double-stranded D-loop is stable even in the absence of the recombinase. Removing the recombinase before isolating the complexes e.g. by binding and thus capturing the complexes to a solid support, may reduce non-specific binding interactions with the solid support and may also reduce the possibility of recombinase interference with probe binding to the support by steric effects.

The proteolytic digest can be terminated by inactivating the proteolytic enzyme. Preferably, a protease inhibitor such as PMSF is used for this purpose. Thus, according to one embodiment, after performing the proteolytic digest the proteolytic enzyme is inactivated, preferably by adding a protease inhibitor.

***Step c)***

In step c), the complexes from which the recombinase may have been removed, are separated from the remaining sequencing library, thereby enriching the target sequences. As discussed above, the invention provides a rapid and efficient method for enriching target sequences containing a region of homology with the invasion probes. Typically, the target sequences are comprised in the sequencing library in a small amount, e.g. in the order of between 2% and 0.0003%. Further embodiments will also be described subsequently. The present invention allows to identify and efficiently enrich even very low-abundance target sequences from the primary sequencing library.

For separating and thus isolating the complexes, several methods are feasible and non-limiting examples are described below.

According to one embodiment, the complexes are isolated by binding them to an appropriate surface of a solid phase. The surface may be functionalized to allow specific binding of the complexes. Here, many isolation methods are feasible which also depend on whether synaptic complexes were/are provided that are labeled with a capture moiety or not.

According to one embodiment, the complexes comprise or are provided with a capture moiety which facilitates the separation of the complexes. Here, several embodiments are feasible and non-limiting examples will be desired in the following. As discussed above, the invasion probes and/or the stabilization probes can be labeled with a capture moiety. Labeling with a capture moiety can be performed prior to forming the complex and is preferably performed during synthesis of the probes as was described above. However, the invasion probe and/or the stabilization probe may also be labeled with a capture moiety after the synaptic complex was formed in step b). In this case, unlabeled invasion probes and optionally unlabeled stabilization probes are used, thereby providing an unlabeled complex. After complex formation, the invasion probe and/or the stabilization probe (if used for complex stabilization) can be extended with labeled nucleotides, e.g. biotinylated nucleotides by a polymerase reaction after hybridization and thus complex formation has occurred. Thereby, again capture moiety labeled complexes are provided. However, it is preferred that labeling with the capture moiety is performed prior to forming the synaptic complexes. Most preferred, the invasion probe is labeled with a capture moiety during synthesis of the probe.

For separation of the complexes wherein e.g. the invasion probe and/or the stabilization probe comprises a capture moiety, a binding agent can be used which binds the capture moiety with high affinity. Suitable capture moieties and binding agents were described above and it is referred to the above disclosure. Preferably, the invasion probe is labeled with biotin, and streptavidin or avidin is used as binding agent. The binding agent can be coupled covalently or non-covalently to a solid support in order to facilitate the separation of the complexes.

According to a further embodiment, unlabeled probes are used and the complexes are captured using a binding agent which specifically binds to the complex and/or a component thereof. Thus, even if no capture moiety like biotin is used for labeling the complex, the unlabeled complex can be separated from non-complexed DNA based on selective binding of a binding agent, which is specific to the synaptic DNA-RecA complex or to the recombinase comprised in the complex. Preferably, the used binding agent specifically binds to the recombinase and thus is an anti-recombinase binding agent. As binding agent, an antibody or a binding fragment thereof can be used. Polyclonal or monoclonal antibodies can be used. Preferably, monoclonal antibodies are used. Using a respective binding agent which e.g. binds to the recombinase for capturing the complex allows to use unlabeled probes. This is cost efficient and furthermore, also allows the amplification of the unmodified probes. Thus, according to one embodiment, the isolation of the complexes involves using a binding agent which specifically binds to the complexes, wherein according to one embodiment, the binding agent is an antibody or fragment thereof which binds the recombinase. Suitable variations of this embodiment are shown in figure 2. When aiming to bind the recombinase for capturing the complex, no proteolytic digest should be performed prior to capturing the complex. The anti-recombinase binding agent can be added either prior to or after complex formation. The anti-recombinase binding agent may also be coupled to a solid support, such as for example magnetic beads, which allows direct capture and thus isolation of the complexes. However, also anti-recombinase binding agents such as anti-recombinase antibodies can be used which are not directly coupled to a solid support. In this embodiment, the anti-recombinase binding agent binds to the complexes in solution and is subsequently captured by a second binding agent, which is suitable of binding and thus capturing the anti-recombinase binding agent and which is coupled to a solid support. As second binding agent which is suitable for specifically binding e.g. an anti-recombinase antibody, Protein A or Protein G can be used, preferably attached to magnetic beads as solid support. Thereby, the anti-recombinase antibody that is bound to the complex can be captured, thereby also capturing the complex.

In one embodiment, the anti-recombinase binding agent is added prior to or during complex formation. In this embodiment, an anti-recombinase binding agent is used which does not inhibit the formation of the D-loop. The anti-recombinase binding agent which preferably is an anti-recombinase antibody will bind to the complexes and can afterwards be captured by a second binding agent, for example by using Protein A or Protein G coated magnetic beads. This embodiment has the advantage that handling steps can be saved. In the respective embodiments wherein an anti-recombinase binding agent such as an anti-recombinase antibody is used, a proteolytic digest as described above can be performed to remove the recombinase from the synaptic complexes, however, after the complexes were bound and accordingly captured to the surface of a solid support such as e.g. magnetic beads.

According to one embodiment, the binding agent which is used for capturing the complexes is coupled to the surface of a solid support, thereby allowing to directly bind and separate the complexes. However, as described above, the binding agent may also comprise, respectively may provide an affinity tag and/or may itself be recognized by a second binding agent, such as for example protein A or protein G in the case of using an antibody as first binding agent which binds to the complexes, respectively a component thereof. According to one embodiment, the binding agent provides a capture moiety.

Therefore, as described above, the complexes may be captured by binding agents that are provided on the surface of a solid support. For example, the surface of the solid support can be functionalized with appropriate binding agents specific for the used capture moiety and/or specific for the complexes, respectively a complex component (such as e.g. the recombinase). Methods for surface functionalization are known in the prior art and thus do not need any further description here. Preferably, the capture moiety is selected from biotin, digoxigenin and haptens and labels the invasion probes and/or stabilization probes, preferably labels the invasion probes. Thus, according to one embodiment, the surface used for binding the complexes is functionalized with an appropriate binding agent for binding the capture moiety, wherein preferably the capture moiety is selected from biotin, digoxigenin and haptens. Preferably, the binding agent is streptavidin or avidin in case of biotin. The solid support may have any form and can be provided by columns, functionalized reaction vessels or wells, particles, filters, fibers, membranes or any other common solid support that can be used in separation technologies. Preferably, magnetic particles are used for providing the surface for binding the complexes. Magnetic particles e.g. having superparamagnetic, paramagnetic, ferromagnetic or ferrimagnetic properties can be easily processed by the aid of a magnet.

After capture of the complexes to the solid support, one or more washing steps can be performed in order to remove non-specifically bound or unbound material. According to one embodiment at least one washing step is performed above room temperature, preferably at a temperature of at least 40°C, such as at least 50°C, at least 55°C, at least 60°C or at least 65°C. However, the temperature during washing should be below the melting point of the formed hybrids. It was found that performing at least one washing step at elevated temperature reduces unspecific binding while preserving the specific binding of the invasion probes to the target sequences. Otherwise, the same washing buffers as used in the prior art may be used. According to one embodiment, at least one washing step is performed at room temperature, followed by one or more, for example at least two or at least three washing steps at elevated temperature as described above. A final washing step with water may be performed.

The captured complexes can then be eluted with a suitable elution solution. As discussed above, the recombinase may have been removed from the complexes prior to separating them from the remaining sample, e.g. by binding the recombinase depleted complexes to a solid support.

According to one embodiment which is particularly feasible if the invasion probe and/or the stabilization probe is provided with a capture moiety such as biotin, the captured complex is denatured during elution, e.g. by adding a base such as NaOH or KOH. Thereby, the captured DNA is rendered single stranded. Depending on the used capture moiety, the probe labeled with the capture moiety may remain bound to the solid support, while the unlabeled strands or nucleic acids are released. The eluted target nucleic acid can be neutralized, if desired.

If the complex comprises the recombinase and is separated and thus captured by using an anti-recombinase binding agent, the captured nucleic acids can be released from the complex by performing a proteolytic digest as described above.

***Further embodiments***

Further non-limiting and preferred embodiments of the present invention will be described in the following.

According to one embodiment, the enriched target sequences are denatured.

The enriched target sequences may optionally be further purified after binding and accordingly after capture to the solid support. For purification, any nucleic acid purification method can be used.

According to one embodiment, two or more target enrichment cycles according to the method of the present invention comprising steps a) to c) described above are performed to increase the enrichment factor. Thereby, the amount of target sequences corresponding to the target region of interest can be increased in the provided target enriched sequencing library. Accordingly, the enriched target sequences and hence the enriched library output obtained after an enrichment cycle may be used as input for performing a further target enrichment cycle. Thereby, the enrichment of target sequences can be increased. Either the same or a different set of invasion probes (and optionally stabilization probes) can be used in each enrichment cycle. According to one embodiment, in sum, 10 enrichment cycles or less, 7 enrichment cycles or less, 5 enrichment cycles or less, 4 enrichment cycles or less or 3 enrichment cycles or less are performed. Furthermore, as will also be described in the following, intermediate steps such as e.g. an amplification step can be performed between two enrichment cycles.

According to one embodiment, an amplification reaction is performed between the individual enrichment cycles. This is e.g. feasible if the primary sequencing library comprises merely a low amount of target sequences, e.g. in the order of 0.05% or less, 0.01% or less, 0.005% or less, 0.001% or less or 0.00075% or less. Furthermore, this is e.g. feasible if the primary sequencing library only comprises very low amounts of DNA such as for example 0.75 $\mu$g or less, in particular 0.5 $\mu$g or less DNA. In said amplification reaction, the enriched target sequences are amplified thereby increasing the amount of target sequences for the subsequent enrichment cycle. However, performing an amplification reaction always poses the risk that wrong nucleotides are incorporated due to a misreading of the polymerase. This can falsify the sequencing results, which is in particular a problem if for example mutations or allelic variations are supposed to be analyzed in the sequencing reaction. Therefore, it is preferred to either perform no amplification reaction between each enrichment cycles or, if an amplification reaction is performed, that e.g. 25 or less, 20 or less, 15 or less, 10 or less, 7 or less or preferably 5 amplification cycles or less are performed in a respective amplification reaction. For performing a respective amplification reaction, primers can be used which hybridize to the adapter sequences which preferably flank the target sequence at its 3' and 5' end (see above regarding the preparation of sequencing libraries comprising adaptors).

The method according to the present invention allows the specific enrichment of target sequences, even low-abundant sequences, from sequencing libraries, thereby providing a target enriched sequencing library that is suitable for next generation sequencing. It is a general aim that a substantial portion of the enriched target sequences lies in the target region of interest. The more enriched sequences lie in the target region of interest, the better is the enrichment result and less sequencing power is spent on sequencing nucleic acids which do not lie in the target region of interest. According to one embodiment, at least 50% of the enriched sequences lie within the target region, preferably at least 55%, at least 60%, at least 65%, more preferably at least 70%. Furthermore, a good read depth and thus coverage can be achieved with the method according to the present invention. As described above, the evenness of coverage across the target region of interest may also be increased if increasing the number of different invasion probes used for enrichment. Suitable embodiments and designs were described above.

In certain embodiments, the adapter-ligated nucleic acids are used without explicit size selection. According to certain embodiments, the method is performed without performing an amplification prior to performing the (first) enrichment using the method of the present disclosure. As described above, if more than one enrichment cycle is performed, an amplification can be performed between two enrichment cycles.

Performing a targeted enrichment has the advantage that non-target sequences are depleted and accordingly, that the sequencing reaction is focused on target sequences which comprise sequences corresponding to the target region of interest. As next generation sequencing allows massive parallel sequencing in one sequencing run, this often has the effect that the sequencing capacity is not exhausted by one target enriched sequencing library. Therefore, it is within the scope of the present invention that after target enrichment, several target enriched sequencing libraries may be combined and subjected to a single sequencing reaction. Respective multiplexing methods wherein several target enriched sequencing libraries are combined and sequenced in parallel in a single run are known in the prior art and were also described above. In order to allow the subsequent assignment of the obtained sequencing results to the individual target enrichment libraries, each target enriched library usually comprises its own unique "index" or "bar-code". As discussed above, specific index adapters may be used in the preparation of the primary sequencing library. Furthermore, it is also possible to introduce index sequences after obtaining the target enriched sequencing library, for example using specific PCR primers which hybridize to the universal adapters of the sequencing library (which do not comprise an index), wherein said PCR primers additionally comprise and thus provide an index sequence, thereby introducing a library specific index during a respective index PCR. If a target enriched sequencing library comprises a library specific index, multiple target enriched sequencing libraries can be combined and sequenced in one run.

Furthermore, the method according to the first aspect of the present invention may comprise

a) massive parallel sequencing of the target sequences comprised in the provided target enriched sequencing library, preferably by the method according to the second aspect of the present invention.

The massive parallel sequencing of the target sequences comprised in the provided target enriched sequencing library by next generation sequencing will be described in further detail in conjunction with the second aspect according to the present invention. It is referred to the respective disclosure which also applies here.

Suitable and preferred applications of the method were already described above. The use/application in particular depends on the chosen target region of interest. Further exemplary applications of the method and target regions of interest are described in the following and include but are not limited to sequencing or resequencing of any arbitrarily defined portion of a previously sequenced reference genome as target region of interest for research or diagnostic purposes; exome- sequencing or resequencing (wherein the exome corresponds to all exons in a genome or to exons from a set of genes of interest, for example genes implicated in cancer or other diseases); promoterome sequencing or resequencing (wherein the promoterome corresponds to all promoters in a genome or promoters from a set of genes of interest, for example genes implicated in cancer or other disease); enhancerome sequencing or resequencing (wherein the enhancerome corresponds to all enhancers in a genome or enhancers from a set of genes of interest, for example genes implicated in cancer of other disease); 5’ or 3’ UTRome sequencing or resequencing; TEZome (transposon exclusion zones) sequencing or resequencing (including epigenetically bivalent domains); transcriptome sequencing or resequencing; bacterial and insect genome assemblies, sequencing of phylogenetically conserved sequences (for example 16S ribosomal RNA); variant discovery by whole-genome resequencing or whole-exome capture; gene discovery in metagenomics; bacterial genome resequencing; DNA methylation analysis, for example one can capture a specific target region of interest and bisulfite resequence the captured sequences; resequencing of CpG islands; resequencing of other sets of distinct genomic features ("omes") that constitute less than 10 %, or less than 5% of the human genome or other complex genomes and/or resequencing of large contiguous genomic regions. Furthermore, viral sequences can be enriched for sequence analysis (for example HIV sequences in random-primed cDNA from patient samples). The method can also be used for somatic mutation detection. This may include e.g. deep resequencing of genes in tumor or non-tumor (normal) samples.

According to one embodiment, the target region of interest may comprise selected genes or all genes located on a specific chromosome, such as e.g. the X- or Y chromosome. The method may also be used for non-invasive prenatal detection of chromosomal aneuploidies such as trisomy 21 or other fetal aneuploidies. For prenatal applications, circulating cell free DNA is isolated preferably from maternal blood samples.

According to one embodiment, the target region of interest comprises or consists of a set of kinases and kinase related genes.

According to one embodiment, the target region of interest is provided by a set of genes that are of interest for a therapeutic or diagnostic application. The target region of interest may also be provided by selected exons or all exons of the genes comprised in the set of genes of interest.

According to one embodiment, the target region of interest may comprise cancer related genes, for example at least 10 cancer related genes, at least 20 cancer related genes or at least 30 cancer related genes. The respective genes that are targeted may include one or more genes that are selected from the group ABL1, JAK2, AKT1, JAK3, ALK, KIT, AR, KRAS, ATM, MAP2K1, BRAF, MAP2K4, CDKN2A, MET, CSF1R, NOTCH1, CTNNB1, NPM1, EGFR, NRAS, ERBB2, PDGFRA, ERBB4, PIK3CA, FANCA, PIK3R1, FANCC, PTEN, FANCF, RET, FANCG, RUNX1, FGFR1, SMAD4, FGFR2, SMO, FGFR3, SRC, FLT3, STK11, HRAS, TP53, IDH1, VHL, IDH2, WT1 and MAP2K2. As described above, the target region of interest may also be provided by selected exons or all exons of the genes comprised in the set of genes of interest.

According to one embodiment, the target region of interest may comprise genes that are associated with cardiomyopathy and may comprise at least 5 genes, at least 10 genes, at least 20 genes or at least 30 genes. The targeted genes are associated with cardiomyopathy, such as hypertrophic cardiomyopathy, dilated cardiomyopathy, and arrythmogenic right ventricular cardiomyopathy. The targeted genes may include one or more genes selected from the group TTR, ACTC1, DES, RBM20, MYL2, TNNI3, LMNA, TGFB3, MYL3, TPM1, SGCD, DSP, MYOZ2, TTN, VCL, PKP2, NEXN, ACTN2, LDB3, DSG2, MYH6, CSRP3, ABCC9, DSC2, MYH7, PLN, SCN5A, TMEM43, MYBPC3, TNNC1, TAZ, JUP, TNNT2 and TCAP. As described above, the target region of interest may also be provided by selected exons or all exons of the genes comprised in the set of genes of interest.

Similarly, the target region of interest may comprise or consist of genes associated with arrhythmia (e.g. the targeted genes may include one or more genes or their exons selected from the group KCNQ1, CAV3, SCN1B, KCNH2, SCN4B, KCNE3, KCNJ2, AKAP9, SCN3B, ANK2, SNTA1, RYR2, KCNE1, SCN5A, KCNJ2, KCNE2, GPD1L, CASQ2, CACNA1C, CACNB2), Noonan syndrome and related disorders such as LEOPARD, cardio-facio-cutaneous syndrome and Costello syndromes (e.g. the targeted genes may include one or more genes or their exons selected from the group BRAF, MAP2K2, RAF1, CBL, NRAS, SHOC2, HRAS, PTPN11, SOS1, MAP2K1, KRAS, NF1, SPRED1), Connective Tissue Disorders, such as Marfan syndrome, Ehlers-Danlos syndrome, Loeys-Dietz syndrome, thoracic aortic aneurysm and dissection (TAAD), Stickler syndrome, Osteogenesis imperfecta and other related disorders (e.g. the targeted genes may include one or more genes or their exons selected from the group AMPD1, COL6A2, TCAP, LMNA, DES, SGCB, SEPN1, DYSF, TPM2, TPM3, COL6A3, FKTN, ACTA1, EMD, POMT1, POMGNT1, DMD, TRIM32, ANO5, FHL1, FKRP, PYGM, ITGA7, TNNT1, TNNI2, ISPD, MYOT, CAPN3, SGCE, SGCD, CAV3, LAMA2, SIL1, CHKB, POMT2, PLEC, LARGE, SGCA, SGCG, COL6A1). As described above, the target region of interest may also be provided by selected exons or all exons of the genes.

Furthermore, the target region of interest may comprise genes associated with neurological diseases and disorders, including Parkinson's disease, Alzheimer's disease, epilepsy, autism and schizophrenia. Other diseases include aortopathies, multiple scleroses (MS), cardiovascular diseases and/or different forms of cancer. As described above, the target region of interest may also be provided by selected exons or all exons of the genes comprised in the set of genes of interest.

Furthermore, the target region of interest may comprise sequences of the Major Histocompatibility Complex. MHC has been shown to play a critical role in the development or progression of hundreds of diseases, including cancers, AIDS, diabetes, arteriosclerosis and leukemia. Given its integral function in the regulation of immune system, MHC has become a key target in drug research and development for a number of diseases.

Next generation sequencing of bisulfite converted DNA may be used to investigate DNA methylation profiles at a genome-wide scale. Here, bisulfite-converted next generation sequencing libraries are prepared, which are enriched for the coding and regulatory regions of different genes of interest as target region of interest, in particular the coding and/or the regulatory regions. This allows e.g. the quantification of methylation levels of CpG sides in the selected gene.

As described, according to one embodiment, the selected genes of interest are genes involved in a disease. According to another embodiment the selected genes of interest are genes that are not involved in a disease. Such genes may be involved in a biological pathway or process. In other embodiments, the target sequences to be enriched comprise a set of cDNAs or viral sequences. As described above, the target region of interest may also be provided by selected exons or all exons of the genes comprised in the set of genes of interest.

In certain embodiments, the target region of interest corresponds to substantially all or all exons in a genome. However, the target region of interest can include only a portion of the exons in a genome, such as greater than 0.1% of genomic exons, greater than 1% of genomic exons, greater than 10% of genomic exons, greater than 20% of genomic exons, greater than 30% of genomic exons, greater than 40% of genomic exons, greater than 50% of genomic exons, greater than 60% of genomic exons, greater than 70% of genomic exons, greater than 80% of genomic exons, greater than 90% of genomic exons, or greater than 95% of genomic exons. According to one embodiment, the target region of interest comprises or consists of exons from selected genes of interest. The number of exons comprised in respectively defining the target region of interest may be at least 50 exons, at least 75 exons, at least 100 exons, at least 150 exons, at least 200 exons, at least 250 exons, at least 500 exons, at least 750 exons, at least 1000 exons, at least 1500 exons, at least 2000 exons or at least 5000 exons.

As described above, the target region of interest may only correspond to a small fraction of the total DNA such as total genomic DNA. It may e.g. correspond to less than 1%, less than 0.5%, less than 0.25%, less than 0.1%, less than 0.05% or less than 0.01% of the DNA, such as genomic DNA or cDNA. According to one embodiment the DNA is or is derived from genomic DNA and the target region of interest includes a more significant fraction of the total genomic DNA, such that it includes at least about 2% of genomic DNA, about 3% of genomic DNA, about 4% of genomic DNA, about 5% of genomic DNA, about 6% of genomic DNA, about 7% of genomic DNA, about 8% of genomic DNA, about 9% of genomic DNA, about 10% of genomic DNA, or more than 10% of genomic DNA. In some embodiments, the target region of interest which accordingly comprises the target sequences may include more than 10%, more than 20%, more than 50% or essentially all of the genome. Such embodiments may be used to select target sequences from a complex mixture of genomes or a metagenome. Examples of applications of such embodiments include but are not limited to the selection of the DNA from one species from a sample containing the DNA from other species.

In some embodiments, the target region of interest comprises one or more large genomic regions that together span more than or less than 1 Mb. According to certain embodiments, the target region of interest comprises 5 Mb or more, 10 Mb or more, 25Mb or more, 50Mb or more or 100Mb or more of the genome.

Particularly preferred embodiments of the method according to the present invention are described in the following:

According to a first particularly preferred embodiment of the method according to the first aspect of the present invention, a method is provided for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adaptors, wherein the method comprises:

a) providing nucleoprotein filaments comprising

(i) a single-stranded invasion probe, wherein the invasion probe has a region of substantial complementarity to one strand of a double-stranded target sequence,
(ii) a RecA like recombinase;

wherein the nucleoprotein filaments were provided using a plurality of different invasion probes and wherein said invasion probes differ in their region of complementarity to the target region or interest and wherein, preferably, the invasion probes have a length that lies in a range of 15 to 100nt, more preferred 25 to 60nt;

b) forming a complex between an invasion probe and a complementary portion of a target sequence wherein complex formation is mediated by the RecA like recombinase and wherein a plurality of complexes are formed and wherein the formed complexes are stabilized by adding single-stranded stabilization probes which hybridize to the displaced strands of the double-stranded target sequences, whereby double-stranded D-loops are formed, wherein, preferably the stabilization probes are shorter than the corresponding invasion probes;

c) separating the complexes from the remaining sequencing library, thereby enriching the target sequences and providing a target enriched sequencing library.

Preferably, the sequencing library used in the first particularly preferred embodiment comprises the double stranded nucleic acid molecules in an overall amount of 2μg or less, 1μg or less, 0.75μg or less, 0.5μg or less, 0.25μg or less or 0.1μg or less. Preferably, the double stranded nucleic acid molecules comprised in the sequencing library are provided by from fragmented genomic DNA. Preferably, complex formation is terminated and the recombinase is removed from the complex by performing a proteolytic digest using a proteolytic enzyme, preferably proteinase K, and optionally a detergent.

According to a second particularly preferred embodiment of the method according to the first aspect of the present invention, a method is provided for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adaptors, wherein the method comprises:

a) providing nucleoprotein filaments comprising

(i) a single-stranded invasion probe, wherein the invasion probe has a region of substantial complementarity to one strand of a double-stranded target sequence,

(ii) a RecA like recombinase;

wherein the nucleoprotein filaments were provided using a plurality of different invasion probes and wherein said invasion probes differ in their region of complementarity to the target region or interest and wherein the invasion probes have a length that lies in a range of 15 to 100nt, preferably 25 to 60nt and wherein the invasion probes are labeled with a capture moiety, preferably biotin;

b) forming a complex between an invasion probe and a complementary portion of a target sequence wherein complex formation is mediated by the RecA like recombinase and wherein a plurality of complexes are formed and wherein the formed complexes are stabilized by adding single-stranded stabilization probes which hybridize to the displaced strands of the double-stranded target sequences, whereby double-stranded D-loops are formed and wherein the complex formation is terminated and the recombinase is removed from the complex preferably by performing a proteolytic digest using a proteolytic enzyme and optionally additionally using a detergent;

c) separating the recombinase depleted complexes from the remaining sequencing library using a solid phase which is functionalized with a binding agent that specifically binds the capture moiety, thereby enriching the target sequences and providing a target enriched sequencing library.

After separation, the target sequences can be eluted from the solid phase. Furthermore, as described above, washing steps can be performed prior to elution. Suitable embodiments were described above.

According to a third particularly preferred embodiment of the method according to the first aspect of the present invention, a method is provided for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adaptors, wherein the method comprises:

a) providing nucleoprotein filaments comprising

(i) a single-stranded invasion probe, wherein the invasion probe has a region of substantial complementarity to one strand of a double-stranded target sequence,
(ii) a RecA like recombinase;

wherein the nucleoprotein filaments were provided using a plurality of different invasion probes and wherein said invasion probes differ in their region of complementarity to the target region or interest and wherein the invasion probes have a length that lies in a range of 15 to 100nt, preferably 25 to 60nt and wherein the invasion probes are not labeled with a capture moiety;

b) forming a complex between an invasion probe and a complementary portion of a target sequence wherein complex formation is mediated by the RecA like recombinase and wherein a plurality of complexes are formed and wherein the formed complexes are stabilized by adding single-stranded stabilization probes which hybridize to the displaced strands of the double-stranded target sequences, whereby double-stranded D-loops are formed,

c) separating the complexes from the remaining sequencing library using a solid phase which is functionalized with a binding agent that specifically binds to the recombinase, thereby enriching the target sequences and providing a target enriched sequencing library.

According to a fourth particularly preferred embodiment of the method according to the first aspect, a method is provided for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adapters, wherein the method comprises:

a) providing nucleoprotein filaments comprising

(i) a single stranded invasion probe, wherein the invasion probe has a region of substantial complementarity to one strand of a double-stranded target sequence,
(ii) a recombinase;

wherein a plurality of different invasion probes are used and wherein the invasion probes differ in their region of complementarity to the target region of interest

b) forming complexes between the invasion probes and a complementary portion of the target sequences wherein complex formation is mediated by the recombinase, wherein preferably, the formed complexes are stabilized by adding single-stranded stabilization probes which hybridize to displaced strands of the double-stranded target sequences, whereby double-stranded D-loops are formed;

c) separating the complexes from the remaining sequencing library, thereby enriching the target sequences,

wherein two or more cycles of enrichment comprising steps a) to c) are performed and wherein an amplification reaction is performed between the individual enrichment cycles to amplify enriched target sequences prior to performing the next enrichment cycle, wherein for amplification primers are used which hybridize to the adapters.

As described above, e.g. at least 100, at least 200, at least 500, at least 750, at least 1000, at least 2000 or at least 5000 different invasion probes can be used. The enriched target sequences cover the target region of interest, thereby allowing to subsequently sequence the target region of interest. As described above, preferably, a corresponding set of stabilization probes is used. According to one embodiment, the invasion probes have a length that lies in a range of 15 to 100nt, preferably 25 to 60nt and the invasion probes are labeled with a capture moiety, preferably biotin. Details regarding the probe design and the subsequent separation and further processing of the complexes were described above and it is referred to the respective disclosure.

Suitable and further preferred embodiments, in particular with respect to the adaptors, the invasion probes, the stabilization probes, the binding agents, the complex treatment and separation and the solid supports were described above and it is referred to the above disclosure which also applies to the first, second, third and fourth particular preferred embodiment. Furthermore, also further options were described such as performing several enrichment cycles and/or amplification reactions between or after individual enrichment cycles. It is again referred to the above disclosure.

According to a second aspect, a method for sequencing a target region of interest is provided, comprising:

a) providing a sequencing library suitable for massive parallel sequencing and comprising a plurality of double stranded nucleic acid molecules, wherein a portion of the double stranded nucleic acid molecules comprised in the sequencing library, the target sequences, comprise a sequence which lies in the target region of interest;

b) enriching target sequences corresponding to the target region of interest according to the method according to the first aspect of the present invention, thereby providing a target enriched sequencing library; and

c) sequencing the enriched target sequences in parallel.

As discussed above, sequencing is performed on a next generation sequencing platform. All NGS platforms share a common technological feature, namely the massively parallel sequencing e.g. of clonally amplified or single DNA molecules that are spatially separated in a flow cell or by generation of an oil-water emulsion. Massively parallel sequencing in particular refers to performing at least thousands (e.g. at least 50 000), at least 500 000 or at least 1 000 000 sequencing reactions in parallel per run. As described in the background, NGS allows thousands to billions of sequencing reactions to be performed simultaneously. In NGS, sequencing is performed by repeated cycles of polymerase-mediated nucleotide extensions or, in one common format, by iterative cycles of oligonucleotide ligation. After obtaining the target enriched sequencing library using the method according to the present invention, clonal separation of single molecules and subsequent amplification is performed by in vitro template preparation reactions like emulsion PCR (pyrosequencing from Roche 454, semiconductor sequencing from Ion Torrent, SOLiD sequencing by ligation from Life Technologies, sequencing by synthesis from Intelligent Biosystems), bridge amplification on the flow cell (e.g. Solexa/Illumina), isothermal amplification by Wildfire technology (Life Technologies) or rolonies/nanoballs generated by rolling circle amplification (Complete Genomics, Intelligent Biosystems, Polonator). Sequencing technologies like Heliscope (Helicos), SMRT technology (Pacific Biosciences) or nanopore sequencing (Oxford Nanopore) allow direct sequencing of single molecules without prior clonal amplification. Suitable NGS methods and platforms that can be used were also described in the background of the present invention and it is referred to the respective disclosure. The sequencing can be performed on any of the respective platforms using the target enriched sequencing library obtained according to the teachings of the present invention.

The obtained sequence information is aligned to provide the sequence of the target region. Here, methods known in the prior art can be used. Suitable methods are e.g. reviewed in Metzker, 2010.

As discussed above, the enriched target sequences cover the target region of interest, thereby allowing to subsequently sequence the target region of interest. As discussed above, preferably at least 45%, at least 50%, preferably at least 55%, more preferred at least 60% of the sequenced sequences lie within the target region.

According to a third aspect the present invention pertains to the use of the method according to the second aspect for exome sequencing, exon sequencing, targeted genomic resequencing, gene panel oriented targeted genomic resequencing, transcriptome sequencing and/or molecular diagnostics. Further applications and uses were described above and it is referred to the respective disclosure which also applies to the third aspect of the present invention.

According to a fourth embodiment, a kit is provided for performing a method according to the first aspect of the present invention. Said kit comprises:

As component a), adaptors for creating a sequencing library suitable for massive parallel sequencing.
Suitable and preferred adaptors and adaptor lengths were described above in conjunction with the method according to the first aspect of the present invention. It is referred to the respective disclosure. According to one embodiment, the adaptors are index adaptor as described above.

As optional component b), one or more ligation reagents for coupling the adaptors to a nucleic acid fragment. E.g. enzymes such as ligases can be used as ligation reagents. Respective ligation reagents are used in the prior art for preparing next generation sequencing libraries.

As component c), a recombinase, preferably a RecA like recombinase. Suitable and preferred embodiments were described above in conjunction with the method according to the first aspect of the present invention. It is referred to the respective disclosure.

As component d), a non-hydrolyzable co-factor for the recombinase, preferably adenosine 5'-(gamma-thio)triphosphate. Suitable and preferred embodiments were described above in conjunction with the method according to the first aspect of the present invention. It is referred to the respective disclosure.

As component e), a plurality of different invasion probes wherein the invasion probes differ in their region of complementarity to a target region of interest. Suitable and preferred embodiments of the invasion probes, the design of the invasion probes, sets of invasion probes, the invasion probe length and also preferred characteristics of the target regions of interest were described above in conjunction with the method according to the first aspect of the present invention. It is referred to the respective disclosure which also applies here. The plurality of different invasion probes are designed to allow enrichment of target sequences corresponding to a target region of interest and suitable examples of target regions of interest were described above. The sequence composition of the set of invasion probes determines the target sequences that are selected from the sequencing library. The enriched target sequences cover the target region of interest, thereby allowing to subsequently sequence the target region of interest. The plurality of invasion probes may be e.g. designed to target specific gene panels also referred to as gene sets, e.g. gene panels indicative for a specific disease or may be designed to target the exome, the transcriptome or portions thereof.
Preferably, the invasion probes are labeled with a capture moiety, suitable and preferred embodiments were described above and it is referred to the respective disclosure. Preferably, biotin is used as capture moiety.

As component f), a plurality of different stabilization probes being at least partially complementary to the plurality of invasion probes. Suitable and preferred embodiments of the stabilization probes were described above in conjunction with the method according to the first aspect of the present invention. It is referred to the respective disclosure.

As component g), a solid support suitable for capturing synaptic complexes formed between the invasion probes and target sequences. Suitable and preferred embodiments of the solid support were described above in conjunction with the method according to the first aspect of the present invention. It is referred to the respective disclosure. Preferably, the surface of the solid support is functionalized with a binding agent, which specifically binds to the complex. The binding agent may e.g. bind the capture moiety of the invasion probes or may bind to the complexes, such as e.g. the recombinase. Suitable and preferred embodiments of the binding agents were described above in conjunction with the method according to the first aspect of the present invention. It is referred to the respective disclosure.

According to one embodiment, the recombinase and the invasion probes are comprised in the kit as nucleoprotein filaments. This has the advantage that the nucleoprotein filaments are basically ready to be used and can be contacted with the sequencing library from which the target sequences are supposed to be enriched. This saves handling steps for the customer.

The kit may optionally comprise further components and reagents selected from the group of enzymes, reaction buffer for the recombinase, proteolytic enzymes, proteinase inhibitors, detergents, washing solutions, elution solutions, polymerases and amplification reagents.

According to one embodiment, the kit comprises primers which are complementary to a sequence of the adaptors. Said primers can be used, e.g. for amplifying enriched target sequences either prior to sequencing or inbetween enrichment cycles. The primers may also be index primers. If an amplification using index primers is performed inbetween two enrichment cycles (regarding such an amplification inbetween two enrichment cycles see above), this has the advantage that the target sequences and accordingly the target enriched sequencing library would be provided with an index during the enrichment process. This can again safe handling steps. Accordingly, a respective index PCR can also be performed between two enrichment cycles of the method according to the first aspect of the present invention.

This invention is not limited by the exemplary methods and materials disclosed herein. Numeric ranges are inclusive of the numbers defining the range. The headings provided herein are not limitations of the various aspects or embodiments of this invention which can be read by reference to the specification as a whole. According to one embodiment, subject matter described herein as comprising certain steps in the case of methods or as comprising certain ingredients in the case of compositions, solutions and/or materials refers to subject matter consisting of the respective steps or ingredients. It is preferred to select and combine preferred embodiments described herein and the specific subject-matter arising from a respective combination of preferred embodiments also belongs to the present disclosure. The present application claims priority of prior applications US 61/678,818, filed on August 2, 2012, and EP 12 179 098.4, filed on August 2, 2012.

## FIGURES

Figure 1 shows enrichment workflows according to examples 2.1. and 2.2. A. Example 2.1 - target DNA enrichment using invasion probes and stabilization oligonucleotides. B. Example 2.2 -target DNA enrichment using invasion probes, stabilization oligonucleotides and two cycles of enrichment. Boxes with gray background indicate steps of the enrichment workflow. Left arrows indicate first enrichment cycle and right arrows indicate the second enrichment cycle.

**Figure 2** shows further embodiments of the present invention. **A.** shows an enrichment method which is based on the use of biotin/streptavidin for capturing the complexes as is also shown in Figure 1. **B.** to **D.** show variations, which do not require a labeling of the probes. Instead, anti-recombinase binding agents, in the shown embodiment anti-RecA antibodies, are used for capturing the complexes. In the embodiment shown in **B.,** the anti-RecA antibody is added after complex formation and stabilization of the D-Loop by addition of the stabilization probes. The anti-RecA antibodies bind to the complexes and can then be captured on protein A or protein G functionalized surfaces, such as for example coated magnetic beads. Protein A or protein G binds to the anti-RecA antibody which in turn binds to the recombinase and thus the complex. Thereby, the complexes are captured on the surface of the solid support, here magnetic beads. The use of magnetic beads simplifies the handling and allows the easy separation of the solid support with the bound complexes from the remaining sequencing library. After the complexes are captured, a proteolytic digest can be performed in order to destroy proteins comprised in the complex and/or destroy proteins that were used for binding. The protolytic digest can be terminated by inactivating the used protolytic enzyme. For example, a proteinase inhibitor such as PMSF can be added. In the embodiment shown in **B.,** at least one further enrichment cycle is performed. Here, in particular when the sequencing library only comprises minimal amounts of DNA material, it is preferred to perform a PCR amplification prior to performing the next enrichment cycle according to the present invention. After the enrichment has been completed and accordingly, a target enriched sequencing library has been provided, it is an option to pool different target enriched sequencing libraries in order to allow the parallel sequencing of multiple libraries in one run. If the primary sequencing library was not prepared by using index adaptors, respective index sequences can be introduced by performing an index PCR. Details regarding the index PCR were described above, it is referred to the respective disclosure.

In **C.,** a further embodiment is described. Therein, a solid support, here the surface of magnetic beads, is functionalized with an anti-recombinase binding agent such as an anti-RecA antibody. In this embodiment, the complex is thus captured directly to the solid support. The remaining steps are identical to the ones explained for **B..**

In **D.,** the anti-recombinase binding agent, which in this case preferably is a monoclonal antibody, is added directly prior to complex formation. Here, it is important to use an antibody which does not inhibit the D-loop formation. The advantage here is that the antibody is already added at the beginning in one step together with the invasion probes, thereby saving handling steps. The anti-RecA antibody binds to the complexes and can then be captured again with protein A or protein G coated surfaces, such as for example magnetic beads. The remaining steps are again the same.

## EXAMPLES

The following example is provided solely to illustrate the concept of the present invention and not meant to limit the present invention to the embodiments provided.

**Example 1:** Library Construction

Multiple protocols for the preparation of adaptor-ligated genomic DNA libraries are known in prior art. In the following example the library preparation protocol of Illumina, Inc. was used:

1) 3μg human genomic DNA was diluted in 130μl TE and fragmented with the ultrasound device Covaris S220 using following parameters: duty cycle 10%, peak incident power 175 W, cycles per burst 200, time 180 sec, temperature of the water bath 7°C, and power mode frequency sweeping.

2) Sheared DNA was concentrated with 180μl AMPure XP beads (Beckman Coulter). After mixing and 5min incubation at room temperature the magnetic AMPure XP beads were separated and the supernatant was discarded. After two wash steps with 500μl 70% ethanol the beads were air dried for 5 min at 37°C and DNA was eluted with 50μl dd$H_2O$.

3) DNA end repair was carried out by adding 10μl end repair buffer, 1.6μl dNTPs, 1μl T4 DNA polymerase, 2μl Klenow DNA polymerase, 2.2μl polynucleotide kinase and water to a final volume of 100μl. Incubation was carried out for 30 min at 20°C. The 100μl reaction mix was then purified with 180μl AMPure XP beads as described above.

4) After elution with 30μl dd$H_2O$ DNA was treated with 5μl Klenow polymerase, 3μl Klenow (exo -) polymerase and 1μl dATP in a total volume of 50μl for 30 min at 37°C for A-addition.

5) The A-tailed DNA was purified with 90μl AMPure XP beads as described and eluted with 15μl dd$H_2O$. Subsequently, adapter ligation was carried out in a 50μl reaction containing 10μl 5x ligation buffer, 10μl PE adapter oligonucleotide mix:

MPadapter1 5'-GATCGGAAGAGCACACGTCT,
MPadapter2 5'-ACACTCTTTCCCTACACGACGCTCTTCCGATCT

and 1.5μl T4 ligase for 15 min at 20°C.

6) After purification with 90μl AMPure XP beads adapter ligated DNA was enriched by PCR. The reaction mix contained:

15μl adapter ligated DNA
21μl dd$H_2O$,
1.25μl InPE amplification primer 1.0
1.25μl GA Indexing Pre Capture PCR Reverse Primer
10μl 5x Herculase II reaction buffer,
0.5μl 100mM dNTP mix
1μl Herculase II Fusion DNA polymerase.

Cycling conditions were: 98°C, 2 min; 6x(98°C 30 sec, 65°C 30 sec, 72°C 1 min); 72°C 10 min; 4°C forever. The primer sequences were:

InPE amplification primer 1.0
5'-AATGATACGGCGACCACCGAGATCTACACTCTTTCCCTACACGACGCTCTTCCGATCT
Indexing Pre Capture PCR Reverse Primer:
5'-GTGACTGGAGTTCAGACGTGTGCTCTTCCGATCT

7) After purification with 50μl AMPure beads DNA was quantified with Qubit ds Brad-Range Assay Kit and analyzed on an Agilent Bioanalyzer using a DNA 7500 chip.

The average fragment insert size of the library (without adapter sequences) was approximately 200bp.

**Example 2:** Target enrichment

Enrichment experiments were carried out in two different ways (Fig. 1):

- Example 2.1: target enrichment using invasion probes and stabilization oligonucleotides
- Example 2.2: repeated target enrichment using invasion probes and stabilization oligonucleotides (two enrichment cycles)

Due to the similarity of the individual steps in both examples (compare Fig. 1), only the procedure for example 2.2. will be described in detail.

RecA-coated nucleofilaments were prepared by adding 1μl 20μM biotinylated invasion probes (see subsequent Table 1), 2.5μl 10x RecA buffer, 2μg RecA, 5μl 10mM ATPγS to a 20μl final reaction volume. After incubation for 10 min at 37°C, the obtained nucleoprotein filaments (wherein each filament comprises an invasion probe coated with RecA) were added to 4μl gDNA library containing 500ng DNA. The mixture was incubated for 10min at 37°C to form the synaptic complex (triple-stranded D-loop) before adding 1μl 36.5μM stabilizing probe mix (Table 2) for stabilization the complex by providing a double-stranded D-loop. After 5 min incubation at 37°C the reaction was terminated by incubation with 0.5μl proteinase K (20μg/μl) and 1μl 5% SDS for 10 min at 37°C. Finally, the proteinase reaction was stopped by addition of 1μl 100mM proteinase inhibitor PMSF before purification with magnetic MyOne streptavidin C1-beads. 20μl beads were washed 3 times with 100μl 1xB&W buffer and re-suspended in 27.5μl 2xB&W buffer and added to the DNA. For binding DNA and beads were incubated for 30 min at room temperature with shaking (650rpm). After magnetic separation followed by 2 wash steps with 100μl 1 xB&W buffer, each, and 1 wash step with 100μl ddH2O the beads were re-suspended in 50μl 100mM NaOH and incubated for 10 min at room temperature. The supernatant after bead separation containing denatured DNA was transferred to a new tube and neutralized with 16.7μl 330mM HCl and 10μl 200mM Tris-HCl pH8. The single stranded DNA was desalted using a MinElute column (QIAGEN) according to the handbook and eluted with 30μl $ddH_2O$.

A 50μl PCR reaction was set-up, containing 30μl purified DNA, 5.25μl primer MP1 (10μM, 5'-AATGATACGGCGACCACCGAGATCTACACTCTTTCCCTACACGACGCTCTTCCGATCT), 5.25μl primer MP2 (10μM, 5'-GTGACTGGAGTTCAGACGTGTGCTCTTCCGATCT), 10μl 5x Herculase II buffer, 1.125μl dNTP mix (10 mM each) and 1μl Herculase II fusion DNA polymerase. The reaction was cycled 2min 98°C, 18x(98°C 30 sec, 65°C 30 sec, 72°C 1 min), 72°C 10 min and cooled down to 4°C. After purification on a MinElute column and quantitation 500ng enriched library were re-enriched by performing a second enrichment cycle. For this purpose, freshly prepared RecA-nucleofilament and stabilization probes as described above were added. After termination of the complex formation and binding to magnetic beads, purified single stranded DNA was amplified in a post-enrichment index PCR containing 30μl captured single stranded DNA, 10μl 5x Herculase II reaction buffer,1.125 dNTP mix (10mM each), 1μl Herculase II fusion DNA polymerase, 1.25μl primer MP1 (10μM), 1.25μl index primer (e.g. MI12, 10μM, CAAGCAGAAGACG-GCATACGAGATTACAAGGTGACTGGAGTTC), and 5.25μl $ddH_2O$.

The reaction was cycled as described above and purified on a MinElute column according to the manual. After quantitation, quality assessment and adjustment of the DNA concentration and treatment according to manufacturer's protocol, the target-enriched library was sequenced on a Illumina MiSeq machine (50 bp single run).

**Table 1.** Invasion probes for gene DDX31. 5' ends of the oligonucleotides were labelled with a Biotin-TEG (not indicated). In total, 27 invasion probes were used which cover 783 bp of the human genome and were spatially separated by 200nt to 12000nt.

| Invasion Probe | chromosome | Start Pos. | End Pos. | Length | sequence |
|---|---|---|---|---|---|
| LRB_DDX01.p1_01 | chr9 | 135453452 | 135453477 | 26 | TTTGCTGAAGGTCACAGAGTGCATCC |
| LRB_DDX02.p1_01 | chr9 | 135462835 | 135462860 | 26 | AGCTTCGAGCGGCAGAAGTACCTGAG |
| LRB_DDX01.q1_01 | chr9 | 135463293 | 135463318 | 26 | GCATCAAGACTGTCACCCCTCCAAGG |
| DDX006TB.p1_01 | char9 | 135471635 | 135471665 | 31 | CAAGCTTTCACAGTCAGGTTAACAACACACT |
| LRB_DDX03.p1_01 | chr9 | 135472184 | 135472211 | 28 | CCTAAGGACTACTTCAGCAGGACAGTGG |
| LRB_DDX02.q1_01 | chr9 | 135472922 | 135472947 | 26 | CCATCGTTGAAGTGCTGGAGCTTATG |
| LRB_DDX04.p1_01 | chr9 | 135480369 | 135480394 | 26 | GAGGATGCCTTTATGTTGTGGGGAAGGAG |
| LRB_DDX03.q1_01 | chr9 | 135481381 | 135481407 | 27 | TTCCCAAACGCTGTCTTTATGAATAGC |
| LRB_DDX05.p1_01 | chr9 | 135490395 | 135490421 | 27 | AGCGTCGAGAATGTGCAGAAGGAACAG |
| LRB_DDX04.q1_01 | chr9 | 135491359 | 135491388 | 30 | CCCCACTATTGACTTGCTTCCCTTTTATGC |
| LRB_DDX06.p1_01 | chr9 | 135499225 | 135499252 | 28 | GGAGCCATAATGAAGTATCTGGGGGAAC |
| LRB_DDX05.q1_01 | chr9 | 135500378 | 135500409 | 32 | GCAGTGTTGCTGTATGTAATTTTGTCTATGAG |
| DDX074TB.p1_01 | chr9 | 135505567 | 135505597 | 31 | GTCGCTTTTTACAGTGAATGGGCCTTGTAAG |
| LRB_DDX07.p1_01 | chr9 | 135505905 | 135505933 | 29 | GGTTACCCTAACACAATCACAAGGAGAAG |
| LRB_DDX06.q1_01 | chr9 | 135506838 | 135506868 | 31 | TGACGTGAGTCATGGTTATTCACATTTTAG |
| LRB_DDX08.p1_01 | chr9 | 135513854 | 135513880 | 27 | TGACTCCTTCTTGCTCATCCCTACTTC |
| LRB_DDX07.q1_01 | chr9 | 135514811 | 135514836 | 26 | CGCCACAGCCTGATTTCTCTAAAGC |
| LRB_DDX09.p1_01 | chr9 | 135522970 | 135523000 | 31 | ATCATAATGTGGCCTAGTAAATCAAGGAAAC |
| LRB_DDX08.q1_01 | chr9 | 135523968 | 135523996 | 29 | TTCTACTGGCGTGGCCCTGTTTGATTTAC |
| LRB_DDX10.p1_01 | chr9 | 135532161 | 135532193 | 33 | CTTCAACTGGTATAAAGAAAACCTCTCCACTG |
| LRB_DDX09.q1_01 | chr9 | 135533073 | 135533103 | 31 | GTGAGACCAGAAATAGAAAGTGAGGTGACTG |
| LRB_DDX11.p1_01 | chr9 | 135539329 | 135539359 | 31 | AGACTTCACCTGATTTACAGACCCAGGACTC |
| LRB_DDX10.q1_01 | chr9 | 135540843 | 135540870 | 28 | TCTAGCTTTTGTTGGTGCTCTCATAGCC |
| DDX149TB.p1_01 | chr9 | 135543157 | 135543187 | 31 | GCTACATCAGGAGGTCAGTGGGGTGCTTGTG |
| LRB_DDX12.p1_01 | chr9 | 135547955 | 135547984 | 30 | CCGAGAAAGTAAGATGATGAGACCAGTTTGTGG |

(continued)

| Invasion Probe | chromosome | Start Pos. | End Pos. | Length | sequence |
|---|---|---|---|---|---|
| LRB_DDX11.q1_01 | chr9 | 135548929 | 135548958 | 30 | CAGACTTCTTTACATTCCTACCGTCACACC |
| LRB_DDX12.q1_01 | chr9 | 135558674 | 135558705 | 32 | AGGGTAAACTGTAACCACTAAGGAGAAAACTG |

**Table 2.** Stabilization probe oligonucleotides complementary to the displaced target sequence.

| Stabilization Probe | sequence | length |
|---|---|---|
| DDX01.p1a_01 | CACTCTGTGACCTTCAG | 17 |
| DDX02.p1a_01 | GTACTTCTGCCGCTCGA | 17 |
| DDX01.q1a_01 | GGAGGGGTGACAGTCTT | 17 |
| DDX006TB.p1a_01 | TTGTTAACCTGACTGTGA | 17 |
| DDX03.p1a_01 | TCCTGCTGAAGTAGTCC | 17 |
| DDX02.q1a_01 | AGCTCCAGCACTTCAAC | 17 |
| DDX04.p1a_01 | TCCCCACATAAAGGCAT | 17 |
| DDX03.q1a_01 | CATAAAGACAGCGTTTG | 17 |
| DDX05.p1a_01 | CTTCTGCACATTCTCGA | 17 |
| DDX04.q1a_01 | GGGAAGCAAGTCAATAG | 17 |
| DDX06.p1a_01 | CCAGATACTTCATTATG | 17 |
| DDX05.q1a_01 | ACAAAATTACATACAGC | 17 |
| DDX074TB.p1a_01 | CCCATTCACTGTAAAAA | 17 |
| DDX07.p1a_01 | TTGTGATTGTGTTAGGG | 17 |
| DDX06.q1a_01 | TGTGAATAAACCATGAC | 17 |
| DDX08.p1a_01 | GGGATGAGCAAGAAGGA | 17 |
| DDX07.q1a_01 | AGAGAAATCAGGCTGTG | 17 |
| DDX09.p1a_01 | TGATTTACTAGGCCACA | 17 |
| DDX08.q1a_01 | AAACAGGGCCACGCCAG | 17 |
| DDX10.p1a_01 | AGGTTTTCTTTATACC | 17 |
| DDX09.q1a_01 | TCACTTTCTATTTCTGG | 17 |
| DDX11.p1a_01 | GGTCTGTAAATCAGGTG | 17 |
| DDX10.q1a_01 | GAGAGCACCAACAAAAG | 17 |
| DDX149TB.p1a_01 | CCCCACTGACCTCCTGA | 17 |
| DDX12.p1a_01 | ACTGGTCTCATCTTACT | 17 |
| DDX11.q1a_01 | CGGTAGGAATGTAAGA | 17 |
| DDX12.q1a_01 | CTCCTTAGTGGTTACAG | 17 |

**Example 3:** Sequencing results

After sequencing approximately 95% to 97% of the readings were mapped with SMALT (http://www.sanger.ac.uk/resources/software/smalt/) to the human reference genome (hg19) and subsequently analyzed in more detail with the "Hybrid Selection Metrics" software of the Picard tools (http://picard.sourceforge.net). The genomic coordinates of invasion probes were defined as region of design (ROD) or bait region, consisting of 27 oligonucleotides with total size of 783 bp. For definition of the target region of interest (ROI) the region of design was expanded 200 bp upstream and 200 bp downstream from the bait coordinates resulting in a total size of 11490 bp. In particular with the method according to example 2.2, wherein two enrichment cycles were performed, targeted DNA enrichment with 10000-fold to 20000-fold for the region of design were achieved. Furthermore, a good coverage of the target region of interest were achieved with single base resolutions above 20x. The invasion probes used in the experiments cover an extreme small target region with 783 bp, what requires a very strong enrichment efficiency to sequence a significant number of fragments from the target region of the human genome. The need for such high enrichment efficiency, which however, is possible with the present invention, decreases with growing target sizes, resulting in higher percentages of reads which are mappable on the target region. Furthermore, more invasion probes can be used to increase enrichment. Usually, for many applications in praxis the sizes of target regions range between 100 kbp and 60 Mbp for gene panel or whole exome sequencing, respectively. The outstanding capability of the method described in this invention report in combination with its simplicity and speed provides a new standard for targeted DNA enrichment from complex DNA sources.

**Example 4: Protocol modification**

In example 4 an improved washing process was performed which increases the specificity. If not stated otherwise, the reaction conditions were the same as in example 2. RecA-coated nucleofilaments were prepared as described in example 2. The invasion probes were coated with RecA and gamma-S-ATP was added. The mixture was incubated for 10 min at 37°C to allow coating. Afterwards, the adapter ligated sequencing library was added and the mixture was incubated for 10 min at 37°C to form the synaptic complex. Then, single stranded stabilization probes were added to stabilize the D-loop. The mixture was incubated for further 5 min at 37°C. Then, a proteinase K digestion was performed at 37 °C for 10 min as described above. Afterwards, the complexes were isolated using magnetic MyOne streptavidin C1-beads as described above. As described therein, for binding, DNA and beads were incubated for 15 min at room temperature using the B&W buffer of the C1-beads.

Afterwards, several washing steps were performed. After magnetic separation of the beads, one washing step was performed at room temperature using 100μl 1xB&W buffer. Then, three washing steps were performed with 100μl 1x B&W buffer for 5 min at 65°C and one wash step with 100μl $ddH_2O$ at room temperature. Elution and neutralization were again performed as described in example 2. Thereby, a target sequence enriched library was obtained. Afterwards, an enrichment PCR was performed (less than 25 amplification cycles) and the obtained PCR products were purified using the MinElute kit (QIAGEN). The respectively obtained PCR amplicons of the target sequence enriched library were then subjected as input material to a second round of enrichment. Afterwards, the next generation sequencing was performed on the target sequence enriched library that was obtained after the second enrichment cycle.

**Example 5: Comparison of Results obtained with different Embodiments**

Using the enrichment protocol of example 4, a target region of interest was enriched which comprised the ddx31 gene (NGS092A and NGS092B - approx. 169 invasion probes were used for capturing in combination with corresponding stabilization probes). Here, the territory covered by the invasion probes had a length of 5354nt. Thereby, usually a larger target region of interest is covered.

In a further experiment using the technology of example 4 (NGS099 - approx. 313 invasion probes were used for capturing in combination with corresponding stabilization probes), the target region of interest comprised the ddx31 and the EGFR gene. Here, the invasion probes targeted 9962nt. Thereby, a target region substantially larger was captured. Here, also more invasion probes could be used also to improve the coverage of the target region of interest, e.g. by using an overlapping invasion probe design.

In two other experiments, the method according to example 2 (see above) was used to enrich a target region of interest comprising the ddx31 gene (NGS016 and NGS017).

The enrichment factor was calculated by the following formula:

$$\text{Enrichment_Factor} = \frac{\text{Percentage Target Seqs (\%)}^{*}\ \text{Genome size (bp)}}{\text{Target_Size (bp)}^{*}\ 100\%}$$

The subsequent table shows the enrichment factors that were achieved in these experiments:

| Experiment | Enrichment factor |
|---|---|
| NGS016 | 22032 |
| NGS017 | 10209 |
| NGS092A | 106526 |
| NGS092B | 100383 |
| NGS099 | 25574 |

The results show that very high enrichment factors can be achieved with the present invention. As the results show, such enrichment factors are achieved with small target regions of interest (approx. 6kb) as well as with larger target regions of interest (approx. 20kb or 40kb). Hence, an advantage of the method of the present disclosure lies in that it allows to enrich small target regions (for example having a size of 5 kb or less) or larger target regions having a size of at least 5kb, at least 10kb, at least 15kb, at least 25kb, at least 50 kb, at least 100 kb or even larger (see above). In contrast, prior art methods often have the problem that smaller target regions of interest, e.g. having a size of less than 100 kb or less than 50 kb in size are difficult to capture with high specificity. These problems are not seen with the method of the invention.

The higher enrichment factor that can be achieved with the present disclosure has the advantage that less background of unspecific sequences, i.e. less of target sequences, are comprised in the enriched library. Therefore, less sequencing power is lost for sequencing non-target sequences. Depending on the target region of interest, enrichment factors of 10.000, 25.000 and even 100.000 can be achieved as is demonstrated by the examples.

Furthermore, the reproducibility from experiment to experiment having the same set up is high, as can be seen from the comparison of NGS092A and NGS092B, wherein an identical set up was used for capturing ddx31 and EGFR gene. Hence, the experiment-to-experiment reproducibility of target representation in captured sequences is high when using the method of the present disclosure. This is an important advantage in particular for diagnostic applications as the reliability is improved.

## Claims

**1.** A method for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules, wherein the method comprises:

a) providing nucleoprotein filaments comprising

(i) a single stranded invasion probe, wherein the invasion probe has a region of substantial complementarity to one strand of a double-stranded target sequence,

(ii) a recombinase;

b) forming a complex between the invasion probe and a complementary portion of the target sequence wherein complex formation is mediated by the recombinase;
c) separating the complexes from the remaining sequencing library, thereby enriching the target sequences.

**2.** The method according to claim 1, comprising

d) massive parallel sequencing of the target sequences comprised in the provided target enriched sequencing library.

**3.** The method according to claim 1 or 2, **wherein** the double stranded nucleic acid molecules comprised in the sequencing library are flanked by adapters.

**4.** The method according to one or more of claims 1 to 3, **wherein**

(i) the sequencing library comprises the double stranded nucleic acid molecules in an overall amount of 2μg or less, 1.5μg or less, 1μg or less, 0.75μg or less, 0.5μg or less, 0.25μg or less or 0.1μg or less
and/or
(ii) wherein the sequencing library was prepared using 5μg or less, 3μg or less, 2μg or less, 1μg or less, 0.5μg or less or 100ng or less nucleic acid starting material; and/or
(iii) wherein the double stranded nucleic acid molecules comprised in the sequencing library are selected from fragmented genomic DNA or cDNA.

**5.** The method according to one or more of claims 1 to 4, having one or more of the following characteristics:

i) the invasion probes have a length of 150nt or less, 120nt or less or 100nt or less and preferably have a length that lies in a range of 15 to 60 nucleotides;
ii) a plurality of different invasion probes are used and wherein the invasion probes differ in their region of complementarity to the target region of interest;
iii) a plurality of different invasion probes are used and wherein the invasion probes differ in their region of complementarity to the target sequence;
iv) the recombinase is a RecA like recombinase, and preferably is RecA;
v) wherein in step a), the nucleoprotein filaments are prepared by contacting the invasion probes with the recombinase in the presence of a non-hydrolysable co-factor, preferably adenosine 5'-(gamma-thio)triphosphate;
vi) in step b) the complex is stabilized by adding a single-stranded stabilization probe which hybridizes to the displaced strand of the double-stranded target sequence, whereby a double-stranded D-loop is formed, wherein, preferably, the stabilization probe is shorter than the invasion probe;
vii) complex formation is terminated and the recombinase is removed from the complex prior to step c) by performing a proteolytic digest using a proteolytic enzyme, preferably proteinase K; and/or
viii) the separation of the complexes in step c) involves binding the complexes to a surface of a solid support.

**6.** The method according to one or more of claims 1 to 5, **wherein** the complex comprises or is provided with a label to facilitate separation of the complexes in step c), and wherein optionally, the label is a capture moiety allowing to bind the complexes to the surface of a solid support and wherein

(i) the capture moiety is provided by using invasion probes and/or a stabilization probes which comprise a capture moiety;
or
(ii) wherein the complex is provided with a capture moiety by labeling the invasion probes and/or the stabilization probes with a capture moiety after the complex was formed in step b).

**7.** The method according to one or more of the preceding claims, **wherein** the isolation of the complexes involves using a binding agent which specifically binds to the complexes or a component thereof and/or wherein a binding agent is used which binds the capture moiety with high affinity.

**8.** The method according to one or more of claims 1 to 7, **wherein** two or more cycles of enrichment comprising steps a) to c) are performed and wherein optionally, an amplification reaction is performed between the individual enrichment cycles to amplify enriched target sequences prior to performing the next enrichment cycle and wherein preferably, the optional amplification reaction is characterized as follows:

(i) 25 amplification cycles or less, 20 amplification cycles or less, 15 amplification cycles or less, 10 amplification cycles or less or 5 amplification cycles or less are performed in the amplification reaction; and/or
(ii) primers are used for amplification which hybridize to adapters flanking the target sequences.

**9.** The method according to one or more of claims 1 to 8, for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adaptors, wherein the method comprises:

a) providing nucleoprotein filaments comprising

(i) a single-stranded invasion probe, wherein the invasion probe has a region of substantial complementarity to one strand of a double-stranded target sequence,
(ii) a RecA like recombinase;

wherein the nucleoprotein filaments were provided using a plurality of different invasion probes and wherein said invasion probes differ in their region of complementarity to the target region of interest and wherein, preferably, the invasion probes have a length that lies in a range of 15 to 100nt, more preferred 25 to 60nt;
b) forming complexes between an invasion probe and a complementary portion of a target sequence wherein complex formation is mediated by the RecA like recombinase and wherein a plurality of complexes are formed and wherein the formed complexes are stabilized by adding single-stranded stabilization probes which hybridize to the displaced strands of the double-stranded target sequences, whereby double-stranded D-loops are formed, wherein, preferably the stabilization probes are shorter than the corresponding invasion probes; and
c) separating the complexes from the remaining sequencing library using a solid support which is functionalized to be able to specifically bind and thus capture the complexes, thereby enriching the target sequences and providing a target enriched sequencing library.

**10.** The method according to one or more of claims 1 to 9, for enriching target sequences from a sequencing library to provide a target enriched sequencing library, wherein the target sequences to be enriched from the sequencing library comprise a sequence which lies in a target region of interest, wherein the sequencing library is suitable for massive parallel sequencing and comprises a plurality of double-stranded nucleic acid molecules flanked by adapters, wherein the method comprises:

a) providing nucleoprotein filaments comprising

(i) a single stranded invasion probe, wherein the invasion probe has a region of substantial complementarity to one strand of a double-stranded target sequence,
(ii) a recombinase;

wherein a plurality of different invasion probes are used and wherein the invasion probes differ in their region of complementarity to the target region of interest;
b) forming complexes between the invasion probes and a complementary portion of the target sequences wherein complex formation is mediated by the recombinase;
c) separating the complexes from the remaining sequencing library, thereby enriching the target sequences,

wherein two or more cycles of enrichment comprising steps a) to c) are performed and wherein an amplification reaction is performed between the individual enrichment cycles to amplify enriched target sequences prior to performing the next enrichment cycle, wherein for amplification primers are used which hybridize to the adapters.

**11.** The method according to one or more of claims 1 to 10, in particular claim 10, having one or more of the following characteristics:

aa) at least 100, at least 200, at least 500, at least 750, at least 1000, at least 2000 or at least 5000 different invasion probes are used and wherein preferably, additionally corresponding stabilization probes are used;
bb) wherein the sequencing library comprises DNA fragments having a length of 1500bp or less, 1000bp or less, 750bp or less or 500bp or less; and/or
cc) the target region of interest has one or more of the following characteristics:

(i) the target region of interest is a genomic target region;
(ii) the sequencing library is made of genomic DNA and the target region of interest consists of more than 10, more than 25, more than 50, more than 100 or more than 1,000 genomic regions, preferably exons;
(iii) the target region of interest is a set of genes implicated in a disease;
(iv) the target region of interest is provided by a set of genes that are of interest for a therapeutic or diagnostic application or the target region of interest is provided by selected exons or all exons of the genes comprised in said set of genes of interest, and/or
(v) the target region of interest comprises or consists of selected genes or all genes located on a specific chromosome.

**12.** A method for sequencing a target region of interest, comprising:

a) providing a sequencing library suitable for massive parallel sequencing and comprising a plurality of double stranded nucleic acid molecules, wherein a portion of the double stranded nucleic acid molecules comprised in the sequencing library, the target sequences, comprise a sequence which lies in the target region of interest;
b) enriching target sequences corresponding to the target region of interest according to the method of one or more of claims 1 to 11, thereby providing a target enriched sequencing library;
c) sequencing the enriched target sequences in parallel.

**13.** The method according to claim 12, having one or more of the following characteristics:

(i) sequencing is performed on a next generation sequencing platform and wherein preferably, the next generation sequencing platform is selected from a bridge amplification sequencing platform or an emulsion amplification sequencing platform;
(ii) the obtained sequence information is aligned to provide the sequence of the target region; and/or
(iii) the enriched target sequences cover the target region of interest, thereby allowing to subsequently sequence the target region of interest and wherein preferably at least 50%, at least 55% or at least 60% of the sequenced sequences lie within the target region; and/or
(iv) prior to step c),

- two or more target enriched sequencing libraries are combined, wherein the target enriched sequencing libraries comprise library specific index adaptors;

or

- an index PCR is performed to provide a target enriched sequencing library wherein the sequences comprised in the library comprise a library specific index and wherein two or more respectively individually indexed target enriched sequencing libraries are combined;

and wherein the combined target enriched sequencing libraries are sequenced in step c) by massive parallel sequencing.

**14.** Use of a method according to claim 12 or 13, for exome sequencing, exon sequencing, gene panel oriented targeted genomic resequencing, targeted genomic resequencing, transcriptome sequencing, transcript sequencing and/or molecular diagnostics.

**15.** A kit for performing a method according to one or more of claims 1 to 11, comprising:

a) adaptors for creating a sequencing library suitable for massive parallel sequencing;
b) optionally one or more ligation reagents for coupling the adaptors to a nucleic acid fragment;
c) a recombinase, preferably a RecA like recombinase;
d) a non-hydrolyzable co-factor for the recombinase, preferably adenosine 5'-(gamma-thio)triphosphate;
e) a plurality of different invasion probes wherein the invasion probes differ in their region of complementarity to a target region of interest;

f) a plurality of different stabilization probes being at least partially complementary to the plurality of invasion probes; and

g) a solid support suitable for capturing synaptic complexes formed between the invasion probes and target sequences

and wherein optionally, the kit has one or more of the following characteristics:

i) the invasion probes are labeled with a capture moiety, preferably biotin and the surface of the solid support is functionalized with a binding agent which specifically binds to the capture moiety of the invasion probes; and/or

ii) wherein the invasion probes have one or more of the characteristics as defined in claims 5i) to 5iii) and claim 11 aa); and/or

iii) wherein the stabilization probes have one or more of the characteristics as defined in claims 5 vi); and/or

iv) wherein the recombinase and the invasion probes are comprised in the kit as nucleoprotein filaments; and/or

v) wherein the adaptors are index adaptors; and/or

vi) wherein the kit comprises primers which are complementary to a sequence of the adaptors; and/or

vii) wherein the kit comprises further reagents selected from the group of proteolytic enzymes, detergents, a reaction buffer for the recombinase; washing solutions; elution solutions and proteinase inhibitors.

## Patentansprüche

1. Verfahren zum Anreichern von Zielsequenzen aus einer Sequenzierungsbibliothek, um eine Ziel-angereicherte Sequenzierungsbibliothek bereitzustellen, wobei die Zielsequenzen, die aus der Sequenzierungsbibliothek anzureichern sind, eine Sequenz umfassen, die in einem Zielbereich von Interesse liegt, wobei die Sequenzierungsbibliothek für massives paralleles Sequenzieren geeignet ist und eine Vielzahl von doppelsträngigen Nukleinsäuremolekülen umfasst, wobei das Verfahren umfasst:

    a) Bereitstellen von Nukleoproteinfilamenten, umfassend

        (i) eine einzelsträngige Invasionssonde, wobei die Invasionssonde einen Bereich aufweist, der im Wesentlichen komplementär zu einem Strang einer doppelsträngigen Zielsequenz ist,

        (ii) eine Rekombinase;

    b) Bilden eines Komplexes zwischen der Invasionssonde und einem komplementären Teil der Zielsequenz, wobei Komplexbildung durch die Rekombinase vermittelt wird;

    c) Trennen der Komplexe von der verbleibenden Sequenzierungsbibliothek, wodurch die Zielsequenzen angereichert werden.

2. Verfahren nach Anspruch 1, umfassend

    d) massives paralleles Sequenzieren der Zielsequenzen, die in der Ziel-angereicherten Sequenzierungsbibliothek enthalten sind.

3. Verfahren nach Anspruch 1 oder 2, wobei die doppelsträngigen Nukleinsäuremoleküle, die in der Sequenzierungsbibliothek enthalten sind, von Adaptern flankiert sind.

4. Verfahren nach einem oder mehreren der Ansprüche 1 bis 3, wobei

    (i) die Sequenzierungsbibliothek die doppelsträngigen Nukleinsäuremoleküle in einer Gesamtmenge von 2 µg oder weniger, 1,5 µg oder weniger, 1 µg oder weniger, 0,75 µg oder weniger, 0,5 µg oder weniger, 0,25 µg oder weniger oder 0,1 µg oder weniger enthält; und/oder

    (ii) wobei die Sequenzierungsbibliothek unter Verwendung von 5 µg oder weniger, 3 µg oder weniger, 2 µg oder weniger, 1 µg oder weniger, 0,5 µg oder weniger oder 100 ng oder weniger Nukleinsäureausgangsmaterial hergestellt wurde; und/oder

    (iii) wobei die doppelsträngigen Nukleinsäuremoleküle, die in der Sequenzierungsbibliothek enthalten sind, aus fragmentierter genomischer DNA oder cDNA ausgewählt sind.

**5.** Verfahren nach einem oder mehreren der Ansprüche 1 bis 4, das eine oder mehrere der folgenden Eigenschaften aufweist:

i) die Invasionssonden weisen eine Länge von 150 nt oder weniger, 120 nt oder weniger oder 100 nt oder weniger auf und weisen vorzugsweise eine Länge auf, die in einem Bereich von 15 bis 60 Nukleotiden liegt;
ii) eine Vielzahl verschiedener Invasionssonden wird verwendet und wobei die Invasionssonden sich in ihrem Bereich der Komplementarität zum Zielbereich von Interesse unterscheiden;
iii) eine Vielzahl verschiedener Invasionssonden wird verwendet und wobei die Invasionssonden sich in ihrem Bereich der Komplementarität zur Zielsequenz unterscheiden;
iv) die Rekombinase ist eine RecA-ähnliche Rekombinase und ist vorzugsweise RecA;
v) wobei in Schritt a) die Nukleoproteinfilamente durch Kontaktieren der Invasionssonden mit der Rekombinase in der Gegenwart eines nicht hydrolysierbaren Cofaktors, vorzugsweise Adenosin 5'-(gamma-thio)triphosphat, hergestellt werden;
vi) in Schritt b) wird der Komplex stabilisiert, indem eine einzelsträngige Stabilisierungssonde hinzugefügt wird, die an den verdrängten Strang der doppelsträngigen Zielsequenz hybridisiert, wobei eine doppelsträngige D-Schleife gebildet wird, wobei die Stabilisierungssonde vorzugsweise kürzer ist als die Invasionssonde;
vii) Die Komplexbildung wird beendet und die Rekombinase wird vor Schritt c) von dem Komplex entfernt, indem ein proteolytischer Verdau unter Verwendung eines proteolytischen Enzyms, vorzugsweise Proteinase K, durchgeführt wird; und/oder
viii) die Trennung der Komplexe in Schritt c) beinhaltet das Binden der Komplexe an eine Oberfläche eines festen Trägers.

**6.** Verfahren nach einem oder mehreren der Ansprüche 1 bis 5, wobei der Komplex eine Markierung umfasst oder damit versehen wird, um die Trennung der Komplexe in Schritt c) zu erleichtern, und wobei die Markierung optional ein Einfangrest ist, der es ermöglicht, die Komplexe an die Oberfläche eines festen Trägers zu binden, und wobei

(i) der Einfangrest bereitgestellt wird, indem Invasionssonden und/oder Stabilisierungssonden verwendet werden, die einen Einfangrest umfassen; oder
(ii) wobei der Komplex mit einem Einfangrest versehen wird, indem die Invasionssonden und/oder die Stabilisierungssonden mit einem Einfangrest versehen werden, nachdem der Komplex in Schritt b) gebildet wurde.

**7.** Verfahren nach einem oder mehreren der vorhergehenden Ansprüche, wobei das Isolieren der Komplexe die Verwendung eines Bindemittels beinhaltet, das spezifisch an die Komplexe oder eine Komponente davon bindet und/oder wobei ein Bindemittel verwendet wird, das den Einfangrest mit hoher Affinität bindet.

**8.** Verfahren nach einem oder mehreren der Ansprüche 1 bis 7, wobei zwei oder mehrere Anreicherungszyklen, die die Schritte a) bis c) umfassen, durchgeführt werden und wobei optional eine Amplifikationsreaktion zwischen den einzelnen Anreicherungszyklen durchgeführt wird, um angereicherte Zielsequenzen vor dem Durchführen des nächsten Anreicherungszyklus zu amplifizieren und wobei vorzugsweise die optionale Amplifikationsreaktion wie folgt gekennzeichnet ist:

(i) 25 Amplifikationszyklen oder weniger, 20 Amplifikationszyklen oder weniger, 15 Amplifikationszyklen oder weniger, 10 Amplifikationszyklen oder weniger oder 5 Amplifikationszyklen oder weniger werden in der Amplifikationsreaktion durchgeführt; und/oder
(ii) Primer werden zur Amplifikation verwendet, die an Adapter hybridisieren, die die Zielsequenzen flankieren.

**9.** Verfahren nach einem oder mehreren der Ansprüche 1 bis 8 zum Anreichern von Zielsequenzen aus einer Sequenzierungsbibliothek, um eine Ziel-angereicherte Sequenzierungsbibliothek bereitzustellen, wobei die aus der Sequenzierungsbibliothek anzureichernden Zielsequenzen eine Sequenz umfassen, die in einem Zielbereich von Interesse liegt, wobei die Sequenzierungsbibliothek für massives paralleles Sequenzieren geeignet ist und eine Vielzahl von doppelsträngigen Nukleinsäuremolekülen umfasst, die von Adaptern flankiert sind, wobei das Verfahren umfasst:

a) Bereitstellen von Nukleoproteinfilamenten, umfassend

(i) eine einzelsträngige Invasionssonde, wobei die Invasionssonde einen Bereich aufweist, der im Wesentlichen komplementär zu einem Strang einer doppelsträngigen Zielsequenz ist,
(ii) eine RecA-ähnliche Rekombinase;

wobei die Nukleoproteinfilamente unter Verwendung einer Vielzahl von verschiedenen Invasionssonden bereitgestellt wurden und wobei die Invasionssonden sich in ihrem Bereich von Komplementarität zu dem Zielbereich von Interesse unterscheiden und wobei die Invasionssonden vorzugsweise eine Länge aufweisen, die in einem Bereich von 15 bis 100 nt, bevorzugter 25 bis 60 nt, liegt;
b) Bilden von Komplexen zwischen einer Invasionssonde und einem komplementären Teil einer Zielsequenz, wobei Komplexbildung durch die RecA-ähnliche Rekombinase vermittelt wird und wobei eine Vielzahl von Komplexen gebildet werden und wobei die gebildeten Komplexe durch Hinzufügen einzelsträngiger Stabilisierungssonden stabilisiert werden, die an die verdrängten Stränge der doppelsträngigen Zielsequenzen hybridisieren, wobei doppelsträngige D-Schleifen gebildet werden, wobei die Stabilisierungssonden vorzugsweise kürzer sind als die entsprechenden Invasionssonden; und
c) Trennen der Komplexe von der verbleibenden Sequenzierungsbibliothek unter Verwendung eines festen Trägers, der funktionalisiert ist, um Komplexe spezifisch binden und also fangen zu können, wodurch die Zielsequenzen angereichter werden und eine Ziel-angereicherte Sequenzierungsbibliothek bereitgestellt wird.

**10.** Verfahren nach einem oder mehreren der Ansprüche 1 bis 9 zum Anreichern von Zielsequenzen aus einer Sequenzierungsbibliothek, um eine Ziel-angereicherte Sequenzierungsbibliothek bereitzustellen, wobei die aus der Sequenzierungsbibliothek anzureichernden Zielsequenzen eine Sequenz umfassen, die in einem Zielbereich von Interesse liegt, wobei die Sequenzierungsbibliothek für massives paralleles Sequenzieren geeignet ist und eine Vielzahl von doppelsträngigen Nukleinsäuremolekülen umfasst, die von Adaptern flankiert sind, wobei das Verfahren umfasst:

a) Bereitstellen von Nukleoproteinfilamenten, umfassend

(i) eine einzelsträngige Invasionssonde, wobei die Invasionssonde einen Bereich aufweist, der im Wesentlichen komplementär zu einem Strang einer doppelsträngigen Zielsequenz ist,
(ii) eine Rekombinase;

wobei eine Vielzahl verschiedener Invasionssonden verwendet wird und wobei die Invasionssonden sich in ihrem Bereich von Komplementarität zu dem Zielbereich von Interesse unterscheiden;
b) Bilden von Komplexen zwischen den Invasionssonden und einem komplementären Teil der Zielsequenz, wobei Komplexbildung durch die Rekombinase vermittelt wird;
c) Trennen der Komplexe von der verbleibenden Sequenzierungsbibliothek, wodurch die Zielsequenzen angereichter werden,

wobei zwei oder mehrere Anreicherungszyklen, die die Schritte a) bis c) umfassen, durchgeführt werden und wobei eine Amplifikationsreaktion zwischen den einzelnen Anreicherungszyklen durchgeführt wird, um angereicherte Zielsequenzen vor dem Durchführen des nächsten Anreicherungszyklus zu amplifizieren, wobei für die Amplifikation Primer verwendet werden, die an die Adapter hybridisieren.

**11.** Verfahren nach einem oder mehreren der Ansprüche 1 bis 10, insbesondere Anspruch 10, das eine oder mehrere der folgenden Eigenschaften aufweist:

aa) mindestens 100, mindestens 200, mindestens 500, mindestens 750, mindestens 1000, mindestens 2000 oder mindestens 5000 verschiedene Invasionssonden werden verwendet und wobei vorzugsweise zusätzlich entsprechende Stabilisierungssonden verwendet werden;
bb) wobei die Sequenzierungsbibliothek DNA-Fragmente umfasst, die eine Länge von 1500 bp oder weniger, 1000 bp oder weniger, 750 bp oder weniger oder 500 bp oder weniger aufweisen; und/oder
cc) der Zielbereich von Interesse eine oder mehrere der folgenden Eigenschaften aufweist:

(i) der Zielbereich von Interesse ist ein genomischer Zielbereich;
(ii) die Sequenzierungsbibliothek ist aus genomischer DNA und der Zielbereich von Interesse besteht aus mehr als 10, mehr als 25, mehr als 50, mehr als 100 oder mehr als 1000 genomischen Bereichen, vorzugsweise Exons;
(iii) der Zielbereich von Interesse ist ein Satz von Genen, die an einer Krankheit beteiligt sind;
(iv) der Zielbereich von Interesse ist durch einen Satz von Genen bereitgestellt, die für eine therapeutische oder diagnostische Anwendung von Interesse sind, oder der Zielbereich von Interesse wird durch ausgewählte Exons oder alle Exons der Gene bereitgestellt, die in dem Satz von Genen von Interesse enthalten sind, und/oder

(v) der Zielbereich von Interesse umfasst oder besteht aus ausgewählten Genen oder allen Genen, die sich auf einem spezifischen Chromosom befinden.

**12.** Verfahren zum Sequenzieren eines Zielbereichs von Interesse, umfassend:

a) Bereitstellen einer Sequenzierungsbibliothek, die für massives paralleles Sequenzieren geeignet ist und eine Vielzahl von doppelsträngigen Nukleinsäuremolekülen umfasst, wobei ein Teil der doppelsträngigen Nukleinsäuremoleküle, die in der Sequenzierungsbibliothek enthalten sind, die Zielsequenzen, eine Sequenz umfasst, die in dem Zielbereich von Interesse liegt;
b) Anreichern von Zielsequenzen, die dem Zielbereich von Interesse entsprechen, nach dem Verfahren von einem oder mehreren der Ansprüche 1 bis 11, wodurch eine Ziel-angereicherte Sequenzierungsbibliothek bereitgestellt wird;
c) paralleles Sequenzieren der angereicherten Zielsequenzen.

**13.** Verfahren nach Anspruch 12, das eine oder mehrere der folgenden Eigenschaften aufweist:

(i) die Sequenzierung wird auf einer Sequenzierungsplattform der nächsten Generation durchgeführt und wobei die Sequenzierungsplattform der nächsten Generation vorzugsweise ausgewählt ist aus einer Brückenamplifikationssequenzierungsplattform oder einer Emulsionsamplifikationssequenzierungsplattform;
(ii) die erhaltene Sequenzinformation wird ausgerichtet, um die Sequenz des Zielbereichs bereitzustellen; und/oder
(iii) die angereicherten Zielsequenzen decken den Zielbereich von Interesse ab, wodurch ermöglicht wird, dass der Zielbereich von Interesse danach sequenziert wird und wobei vorzugsweise mindestens 50 %, mindestens 55 % oder mindestens 60 % der sequenzierten Sequenzen innerhalb des Zielbereichs liegen; und/oder
(iv) vor Schritt c)

- werden zwei oder mehrere Ziel-angereicherte Sequenzierungsbibliotheken kombiniert, wobei die Ziel-angereicherten Sequenzierungsbibliotheken bibliotheksspezifische Indexadapter umfassen;

oder

- wird eine Index-PCR durchgeführt, um eine Ziel-angereicherte Sequenzierungsbibliothek bereitzustellen, wobei die Sequenzen, die in der Bibliothek umfasst sind, einen bibliotheksspezifischen Index umfassen und wobei zwei oder mehrere jeweils einzeln indexierte Ziel-angereicherte Sequenzierungsbibliotheken kombiniert werden;

und wobei die kombinierten Ziel-angereicherten Sequenzierungsbilbiotheken in Schritt c) durch massives paralleles Sequenzieren sequenziert werden.

**14.** Verwendung eines Verfahrens nach Anspruch 12 oder 13 zum Exomsequenzieren, Exonsequenzieren, genlistenorientierten gezielten genomischen Resequenzieren, gezielten genomischen Resequenzieren, Transkriptomsequenzieren, Transkriptsequenzieren und/oder für molekularer Diagnostik.

**15.** Kit zur Durchführung eines Verfahrens nach einem oder mehreren der Ansprüche 1 oder 11, umfassend:

a) Adapter zum Erzeugen einer Sequenzierungsbibliothek, die für massives paralleles Sequenzieren geeignet ist;
b) optional eines oder mehrere Ligationsreagenzien zum Koppeln der Adapter an ein Nukleinsäurefragment;
c) eine Rekombinase, vorzugsweise eine RecA-ähnliche Rekombinase;
d) ein nicht hydrolysierbarer Cofaktor für die Rekombinase, vorzugsweise Adenosin 5'-(gamma-thio)triphosphat;
e) eine Vielzahl verschiedener Invasionssonden, wobei sich die Invasionssonden in ihrem Bereich der Komplementarität mit einem Zielbereich von Interesse unterscheiden;
f) eine Vielzahl verschiedener Stabilisierungssonden, die mindestens teilweise komplementär zu der Vielzahl von Invasionssonden sind; und
g) ein fester Träger, der zum Fassen synaptischer, zwischen den Invasionssonden und den Zielsequenzen gebildeter Komplexe geeignet ist,

und wobei das Kit optional eine oder mehrere der folgenden Eigenschaften aufweist:

i) die Invasionssonden sind mit einem Einfangrest, vorzugsweise Biotin, markiert und die Oberfläche des festen Trägers ist mit einem Bindemittel funktionalisiert, das spezifisch an den Einfangrest der Invasionssonden bindet; und/oder
ii) wobei die Invasionssonden eine oder mehrere der Eigenschaften aufweisen, wie in Anspruch 5i) bis 5iii) und Anspruch 11aa) definiert; und/oder
iii) wobei die Stabilisierungssonden eine oder mehrere der Eigenschaften aufweisen, wie in Anspruch 5vi) definiert; und/oder
iv) wobei die Rekombinase und die Invasionssonden in dem Kit als Nukleoproteinfilamente umfasst sind; und/oder
v) wobei die Adapter Indexadapter sind; und/oder
vi) wobei das Kit Primer umfasst, die zu einer Sequenz der Adapter komplementär sind; und/oder
vii) wobei das Kit ferner Reagenzien umfasst, die aus der Gruppe von proteolytischen Enzymen, Detergenzien, einem Reaktionspuffer für die Rekombinase;

Waschlösungen; Elutionslösungen und Proteinaseinhibitoren ausgewählt sind.

**Revendications**

1. Procédé d'enrichissement de séquences cibles à partir d'une banque de séquençage pour produire une banque de séquençage enrichie en cible, dans lequel les séquences cibles devant être enrichies à partir de la banque de séquençage comprennent une séquence qui est située dans une région cible d'intérêt, la banque de séquençage étant adaptée pour le séquençage parallèle en masse et comprenant une pluralité de molécules d'acide nucléique double brin, le procédé comprenant :

    a) la fourniture de filaments de nucléoprotéine comprenant

        (i) une sonde d'invasion simple brin, la sonde d'invasion comportant une région de complémentarité substantielle avec un brin d'une séquence cible double brin,
        (ii) une recombinase ;

    b) la formation d'un complexe entre la sonde d'invasion et une partie complémentaire de la séquence cible, la formation de complexes étant médiée par la recombinase ;
    c) la séparation des complexes du reste de la banque de séquençage, de manière à enrichir les séquences cibles.

2. Procédé selon la revendication 1, comprenant

    d) le séquençage parallèle en masse des séquences cibles comprises dans la banque de séquençage enrichie en cible produite.

3. Procédé selon la revendication 1 ou 2, dans lequel les molécules d'acide nucléique double brin comprises dans la banque de séquençage sont flanquées par des adaptateurs.

4. Procédé selon une ou plusieurs des revendications 1 à 3, dans lequel

    (i) la banque de séquençage comprend les molécules d'acide nucléique double brin en une quantité totale de 2 µg ou moins, 1,5 µg ou moins, 1 µg ou moins, 0,75 µg ou moins, 0,5 µg ou moins, 0,25 µg ou moins ou 0,1 µg ou moins

    et/ou

    (ii) dans lequel la banque de séquençage est préparée en utilisant 5 µg ou moins, 3 µg ou moins, 2 µg ou moins, 1 µg ou moins, 0,5 µg ou moins ou 100 ng ou moins de matériau de départ d'acide nucléique ; et/ou
    (iii) dans lequel les molécules d'acide nucléique double brin comprises dans la banque de séquençage sont choisies parmi de l'ADN génomique ou de l'ADNc fragmenté.

5. Procédé selon une ou plusieurs des revendications 1 à 4, ayant une ou plusieurs des caractéristiques suivantes :

i) les sondes d'invasion ont une longueur de 150 nt ou moins, 120 nt ou moins ou 100 nt ou moins et, de préférence, ont une longueur qui est située dans une plage de 15 à 60 nucléotides ;
ii) une pluralité de sondes d'invasion différentes sont utilisées et les sondes d'invasion différant dans leur région de complémentarité avec la région cible d'intérêt ;
iii) une pluralité de sondes d'invasion différentes sont utilisées et les sondes d'invasion différant dans leur région de complémentarité avec la séquence cible ;
iv) la recombinase est une recombinase de type RecA et, de préférence, est RecA;
v) dans lequel, dans l'étape a), les filaments de nucléoprotéine sont préparés par mise en contact des sondes d'invasion avec la recombinase en présence d'un cofacteur non hydrolysable, de préférence l'adénosine 5'-(gamma-thio)triphosphate ;
vi) dans l'étape b), le complexe est stabilisé par ajout d'une sonde de stabilisation simple brin qui s'hybride avec le brin déplacé de la séquence cible double brin, de sorte qu'une boucle D double brin soit formée, dans lequel, de préférence, la sonde de stabilisation est plus courte que la sonde d'invasion ;
vii) la formation de complexes est terminée et la recombinase est enlevée du complexe avant l'étape c) par conduite d'une digestion protéolytique au moyen d'une enzyme protéolytique, de préférence la protéinase K ; et/ou
viii) la séparation des complexes dans l'étape c) met en oeuvre la liaison des complexes à une surface d'un support solide.

**6.** Procédé selon une ou plusieurs des revendications 1 à 5, dans lequel le complexe comprend ou est pourvu d'un marqueur pour faciliter la séparation des complexes dans l'étape c), et dans lequel, facultativement, le marqueur est un fragment de capture permettant la liaison des complexes à la surface d'un support solide et dans lequel

(i) le fragment de capture est fourni en utilisant des sondes d'invasion et/ou des sondes de stabilisation qui comprennent un fragment de capture ; ou
(ii) dans lequel le complexe est pourvu d'un fragment de capture par marquage des sondes d'invasion et/ou des sondes de stabilisation avec un fragment de capture après la formation du complexe dans l'étape b).

**7.** Procédé selon un ou plusieurs des revendications précédentes, dans lequel l'isolement des complexes met en oeuvre l'utilisation d'un agent de liaison qui se lie spécifiquement aux complexes ou un composant de ceux-ci et/ou dans lequel un agent de liaison est utilisé qui se lie au fragment de capture avec une affinité élevée.

**8.** Procédé selon une ou plusieurs des revendications 1 à 7, dans lequel deux ou plus de deux cycles d'enrichissement comprenant les étapes a) à c) sont effectués et dans lequel, facultativement, une réaction d'amplification est conduite entre les cycles d'enrichissement individuels pour amplifier les séquences cibles enrichies avant d'effectuer le cycle d'enrichissement suivant et dans lequel, de préférence, la réaction d'amplification facultative est **caractérisée** comme suit :

(i) 25 cycles d'amplification ou moins, 20 cycles d'amplification ou moins, 15 cycles d'amplification ou moins, 10 cycles d'amplification ou moins ou 5 cycles d'amplification ou moins sont conduits dans la réaction d'amplification ; et/ou
(ii) des amorces sont utilisées pour l'amplification qui s'hybrident avec des adaptateurs flanquant les séquences cibles.

**9.** Procédé selon une ou plusieurs des revendications 1 à 8, pour enrichir des séquences cibles à partir d'une banque de séquençage pour produire une banque de séquençage enrichie en cible, dans lequel les séquences cibles devant être enrichies à partir de la banque de séquençage comprennent une séquence qui est située dans une région cible d'intérêt, dans lequel la banque de séquençage est adaptée pour le séquençage parallèle en masse et comprend une pluralité de molécules d'acide nucléique double brin flanquées par des adaptateurs, le procédé comprenant :

a) la fourniture de filaments de nucléoprotéine comprenant

(i) une sonde d'invasion simple brin, la sonde d'invasion comportant une région de complémentarité substantielle avec un brin d'une séquence cible double brin,
(ii) une recombinase de type RecA ;

les filaments de nucléoprotéine étant fournis en utilisant une pluralité de sondes d'invasion différentes et lesdites sondes d'invasion différant dans leur région de complémentarité avec la région cible d'intérêt et, de préférence, les sondes d'invasion ayant une longueur qui est située dans une plage de 15 à 100 nt, plus préférablement de 25 à 60 nt ;
b) la formation de complexes entre une sonde d'invasion et une partie complémentaire d'une séquence cible, la formation de complexes étant médiée par la recombinase de type RecA et une pluralité de complexes étant formés et les complexes formés étant stabilisés par ajout de sondes de stabilisation simple brin qui s'hybrident avec les brins déplacés des séquences cibles double brin, de sorte que des boucles D double brin soient formées, dans lequel, de préférence, les sondes de stabilisation sont plus courtes que les sondes d'invasion correspondantes ; et
c) la séparation des complexes du reste de de la banque de séquençage au moyen d'un support solide qui est fonctionnalisé de manière à pouvoir se lier spécifiquement et, ainsi, capturer les complexes, de manière à enrichir les séquences cibles et produire une banque de séquençage enrichie en cible.

**10.** Procédé selon une ou plusieurs des revendications 1 à 9, pour enrichir des séquences cibles à partir d'une banque de séquençage pour produire une banque de séquençage enrichie en cible, dans lequel les séquences cibles devant être enrichies à partir de la banque de séquençage comprennent une séquence qui est située dans une région cible d'intérêt, dans lequel la banque de séquençage est adaptée pour le séquençage parallèle en masse et comprend une pluralité de molécules d'acide nucléique double brin flanquées par des adaptateurs, le procédé comprenant :

a) la fourniture de filaments de nucléoprotéine comprenant

(i) une sonde d'invasion simple brin, la sonde d'invasion ayant une région de complémentarité substantielle avec un brin d'une séquence cible double brin,
(ii) une recombinase ;

dans lequel une pluralité de sondes d'invasion différentes sont utilisées et les sondes d'invasion différant dans leur région de complémentarité avec la région cible d'intérêt ;
b) la formation de complexes entre les sondes d'invasion et une partie complémentaire des séquences cibles, la formation de complexes étant médiée par la recombinase ;
c) la séparation des complexes du reste de la banque de séquençage, de manière à enrichir les séquences cibles,

dans lequel deux ou plus de deux cycles d'enrichissement comprenant les étapes a) à c) sont effectués et dans lequel une réaction d'amplification est conduite entre les cycles d'enrichissement individuels pour amplifier les séquences cibles enrichies avant de conduire le cycle d'enrichissement suivant, dans lequel des amorces d'amplification sont utilisées qui s'hybrident avec les adaptateurs.

**11.** Procédé selon une ou plusieurs des revendications 1 à 10, en particulier la revendication 10, ayant une ou plusieurs des caractéristiques suivantes :

aa) au moins 100, au moins 200, au moins 500, au moins 750, au moins 1000, au moins 2000 ou au moins 5000 sondes d'invasion différentes sont utilisées et dans lequel, de préférence, en outre, des sondes de stabilisation correspondantes sont utilisées ;
bb) dans lequel la banque de séquençage comprend des fragments d'ADN ayant une longueur de 1500 pb ou moins, 1000 pb ou moins, 750 pb ou moins ou 500 pb ou moins ; et/ou
cc) la région cible d'intérêt présente une ou plusieurs des caractéristiques suivantes :

(i) la région cible d'intérêt est une région cible génomique ;
(ii) la banque de séquençage est constituée d'ADN génomique et la région cible d'intérêt est constituée de plus de 10, plus de 25, plus de 50, plus de 100 ou plus de 1 000 régions génomiques, de préférence des exons ;
(iii) la région cible d'intérêt est un ensemble de gènes impliqués dans une maladie ;
(iv) la région cible d'intérêt est fournie par un ensemble de gènes qui sont d'intérêt pour une application thérapeutique ou diagnostique ou la région cible d'intérêt est fournie par des exons sélectionnés ou tous les exons des gènes compris dans ledit ensemble de gènes d'intérêt, et/ou
(v) la région cible d'intérêt comprend ou est constituée de gènes sélectionnés ou de tous les gènes situés sur un chromosome spécifique.

**12.** Procédé de séquençage d'une région cible d'intérêt, comprenant :

a) la production d'une banque de séquençage adaptée pour le séquençage parallèle en masse et comprenant une pluralité de molécules d'acide nucléique double brin, dans laquelle une partie des molécules d'acide nucléique double brin comprises dans la banque de séquençage, les séquences cibles, comprennent une séquence qui est située dans la région cible d'intérêt ;
b) l'enrichissement de séquences cibles correspondant à la région cible d'intérêt selon le procédé d'une ou plusieurs des revendications 1 à 11, de manière à produire une banque de séquençage enrichie en cible ;
c) séquençage des séquences cibles enrichies en parallèle.

**13.** Procédé selon la revendication 12, ayant une ou plusieurs des caractéristiques suivantes :

(i) le séquençage est effectuée sur une plate-forme de séquençage de nouvelle génération et, de préférence, la plate-forme de séquençage de nouvelle génération étant choisie parmi une plate-forme de séquençage par amplification en ponts ou une plate-forme de séquençage par amplification en émulsion ;
(ii) les informations de séquence obtenues sont alignées pour fournir la séquence de la région cible ; et/ou
(iii) les séquences cibles enrichies couvrent la région cible d'intérêt, de manière à permettre le séquençage consécutif de la région cible d'intérêt et, de préférence, au moins 50 %, au moins 55 % ou au moins 60 % des séquences séquencées étant situées dans la région cible ; et/ou
(iv) avant l'étape c),

- deux ou plus de deux banques de séquençage enrichies en cible sont combinées, les banques de séquençage enrichies en cible comprenant des adaptateurs d'index de banque spécifique ;

ou

- une PCR d'index est conduite pour produire une banque de séquençage enrichie en cible, les séquences comprises dans la banque comprenant un index de banque spécifique et deux ou plus de deux banques de séquençage enrichies en cible respectivement indexées individuellement étant combinées ;

et dans lequel les banques de séquençage enrichies en cible combinées sont séquencées dans l'étape c) par séquençage parallèle en masse.

**14.** Utilisation d'un procédé selon la revendication 12 ou 13, pour séquençage d'exome, séquençage d'exon, reséquençage génomique ciblé orienté de panel de gènes, reséquençage génomique ciblé, séquençage de transcriptome, séquençage de produits de transcription et/ou diagnostics moléculaires.

**15.** Ensemble pour conduire ou mettre en oeuvre un procédé selon une ou plusieurs des revendications 1 à 11, comprenant ;

a) des adaptateurs pour créer une banque de séquençage adaptée pour le séquençage parallèle en masse ;
b) facultativement, un ou plusieurs réactifs de ligature pour coupler les adaptateurs à un fragment d'acide nucléique ;
c) une recombinase, de préférence une recombinase de type RecA ;
d) un contacteur non hydrolysable pour la recombinase, de préférence l'adénosine 5'-(gamma-thio)triphosphate ;
e) une pluralité de sondes d'invasion différentes, les sondes d'invasion différant dans leur région de complémentarité avec une région cible d'intérêt ;
f) une pluralité de sondes de stabilisation différentes étant au moins partiellement complémentaires de la pluralité de sondes d'invasion ; et
g) un support solide adapté pour capturer des complexes synaptiques formés entre les sondes d'invasion et les séquences cibles

et, facultativement, l'ensemble ayant une ou plusieurs des caractéristiques suivantes :

i) les sondes d'invasion sont marquées avec un fragment de capture, de préférence la biotine et la surface du support solide est fonctionnalisée avec un agent de liaison qui se lie spécifiquement au fragment de capture des sondes d'invasion ; et/ou
ii) dans lequel les sondes d'invasion ont une ou plusieurs des caractéristiques telles que définies dans les revendications 5i) à 5iii) et la revendication 11 aa) ; et/ou

iii) dans lequel les sondes de stabilisation ont une ou plusieurs des caractéristiques telles que définies dans la revendication 5 vi) ; et/ou

iv) dans lequel la recombinase et les sondes d'invasion sont comprises dans l'ensemble en tant que filaments de nucléoprotéine ; et/ou

v) dans lequel les adaptateurs sont des adaptateurs d'index ; et/ou

vi) dans lequel l'ensemble comprend des amorces qui sont complémentaires d'une séquence des adaptateurs ; et/ou

vii) dans lequel l'ensemble comprend des réactifs supplémentaires choisis dans le groupe constitué d'enzymes protéolytiques, de détergents, d'un tampon de réaction pour la recombinase ; de solutions de lavage ; de solutions d'élution et d'inhibiteurs de protéinase.

Fig. 1A

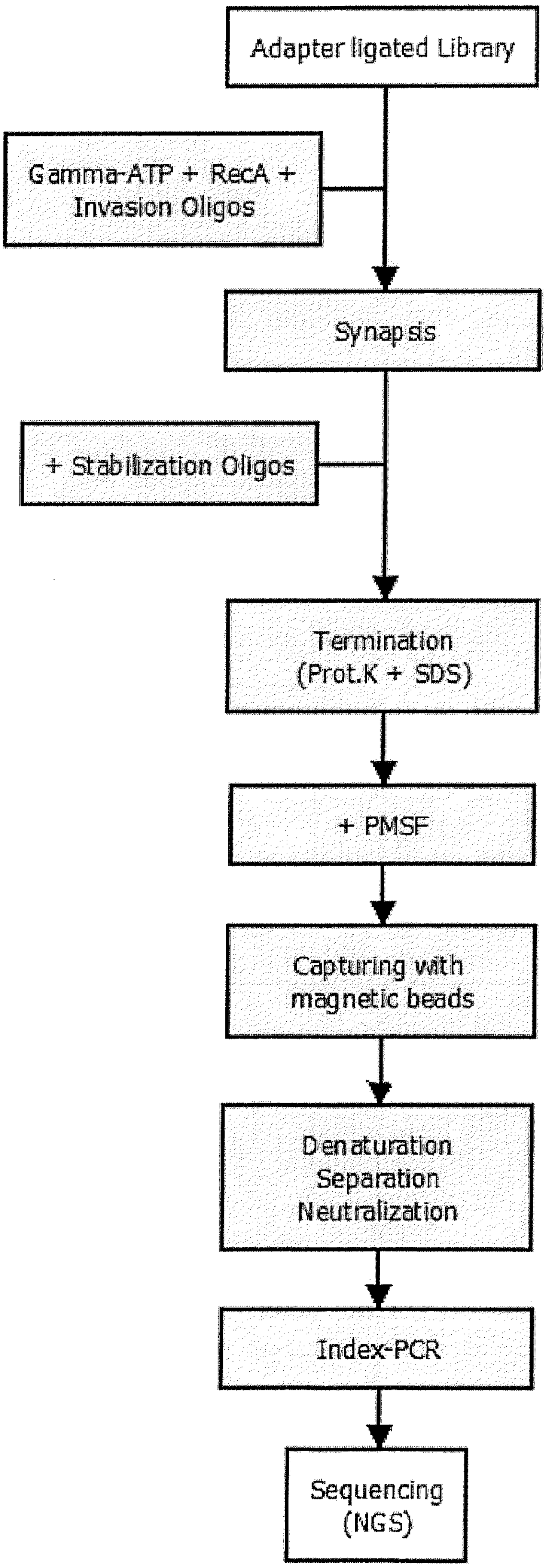
Adapter ligated Library
Gamma-ATP + RecA + Invasion Oligos
Synapsis
+ Stabilization Oligos
Termination (Prot.K + SDS)
+ PMSF
Capturing with magnetic beads
Denaturation Separation Neutralization
Index-PCR
Sequencing (NGS)

Fig. 1B

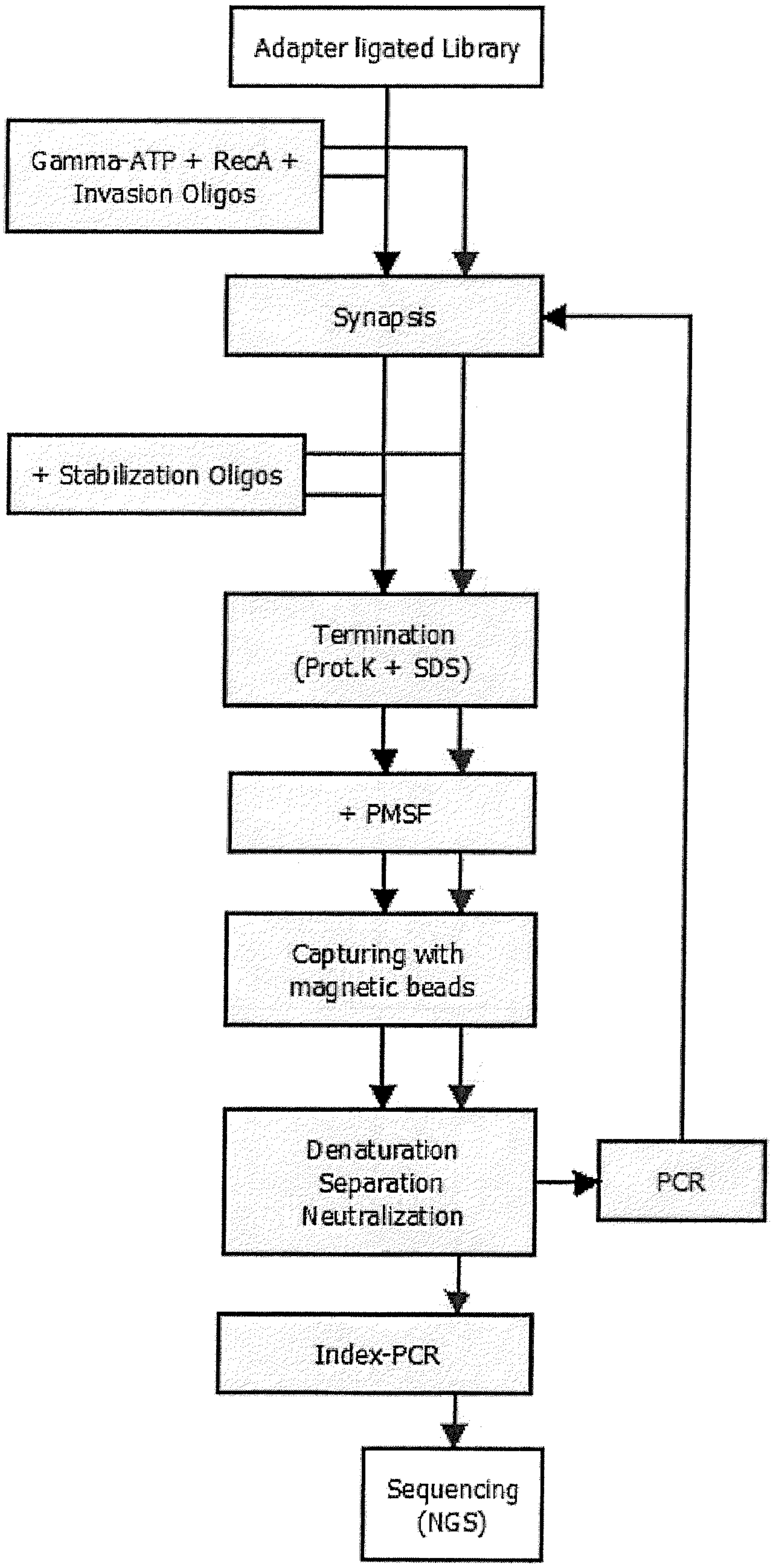
Adapter ligated Library
Gamma-ATP + RecA + Invasion Oligos
Synapsis
+ Stabilization Oligos
Termination (Prot.K + SDS)
+ PMSF
Capturing with magnetic beads
Denaturation Separation Neutralization
PCR
Index-PCR
Sequencing (NGS)

Fig. 2A

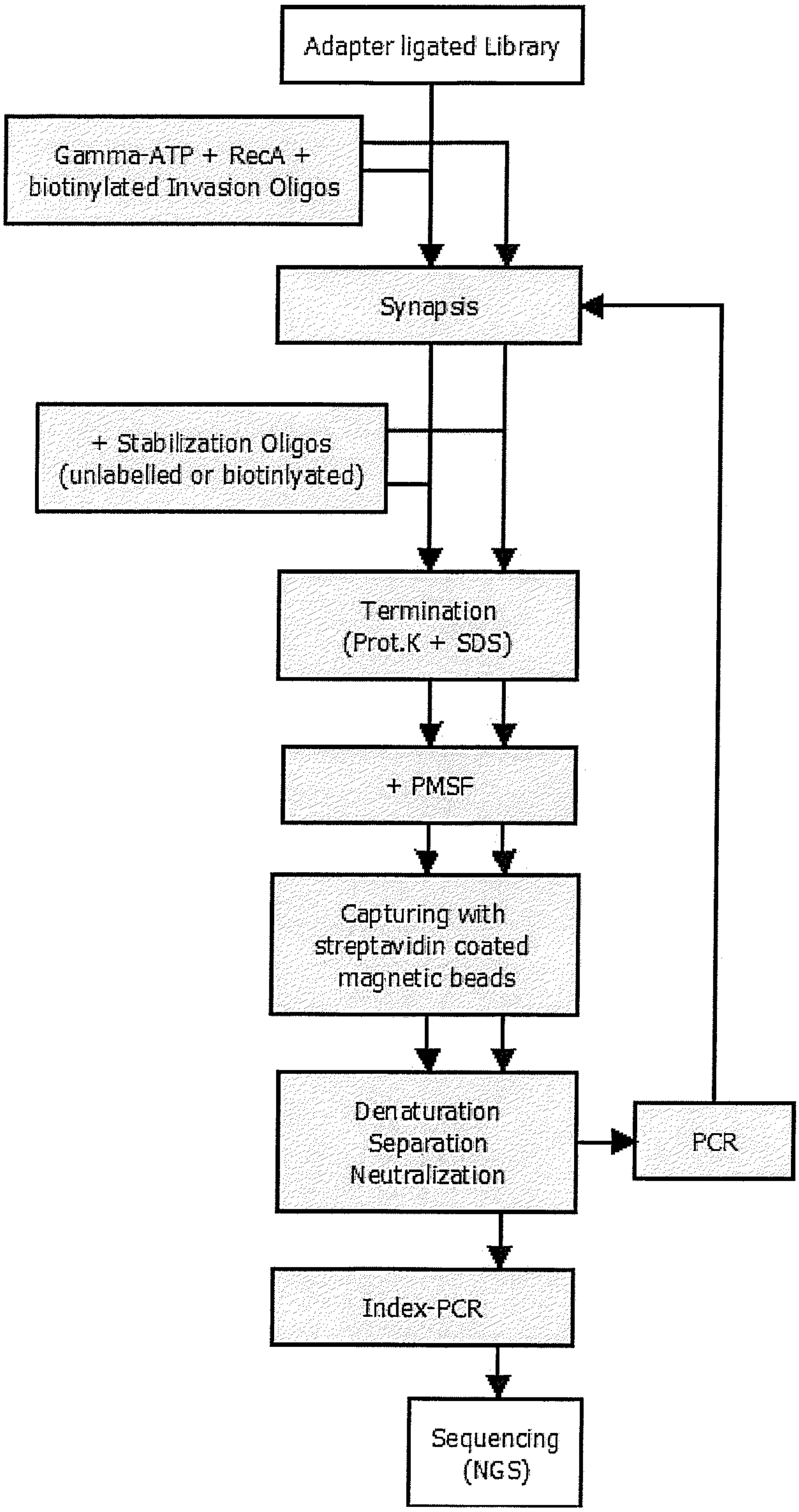
Adapter ligated Library
Gamma-ATP + RecA +
biotinylated Invasion Oligos
Synapsis
+ Stabilization Oligos
(unlabelled or biotinlyated)
Termination
(Prot.K + SDS)
+ PMSF
Capturing with
streptavidin coated
magnetic beads
Denaturation
Separation
Neutralization
PCR
Index-PCR
Sequencing
(NGS)

Fig. 2B

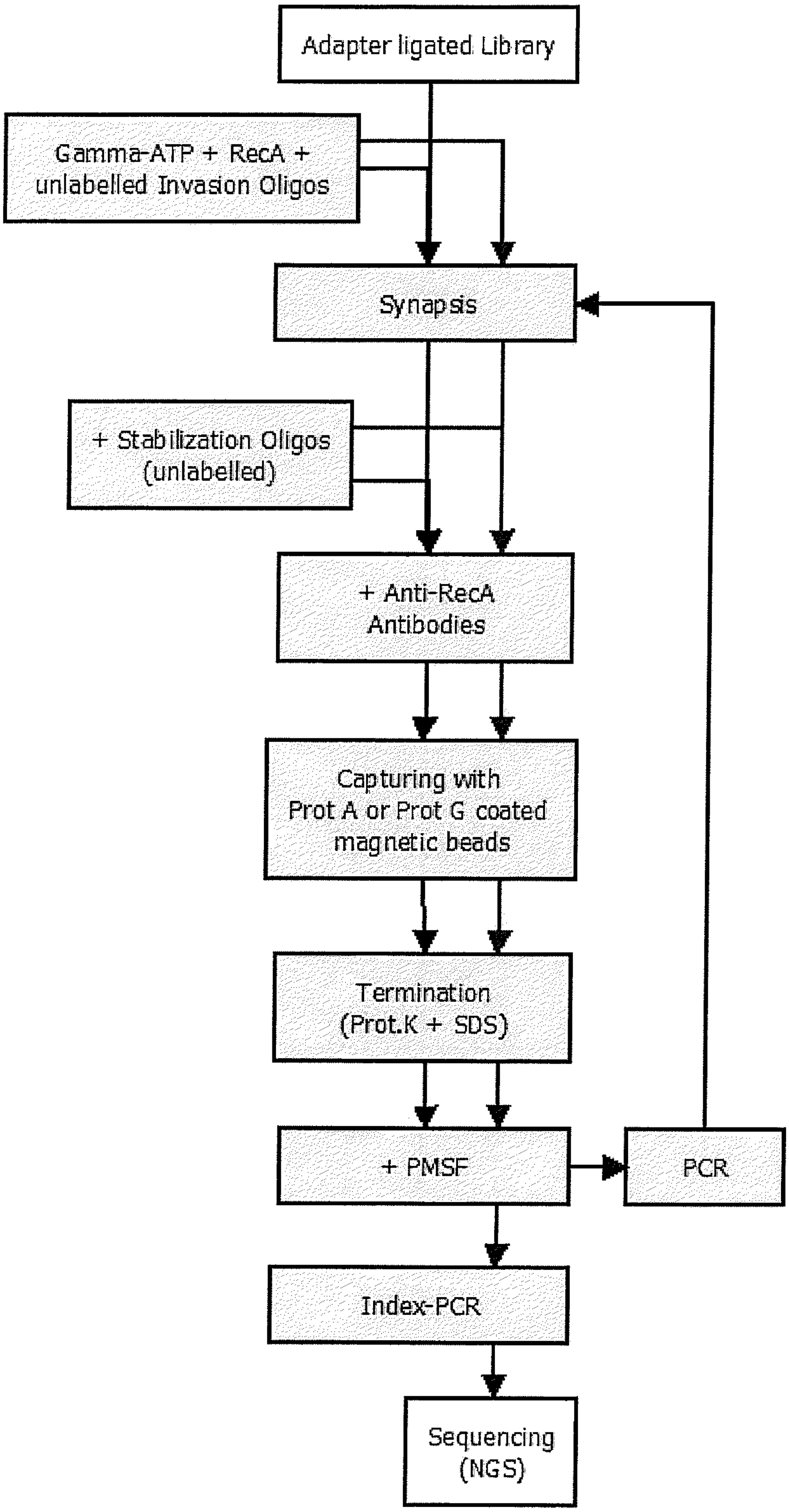
Adapter ligated Library
Gamma-ATP + RecA + unlabelled Invasion Oligos
Synapsis
+ Stabilization Oligos (unlabelled)
+ Anti-RecA Antibodies
Capturing with Prot A or Prot G coated magnetic beads
Termination (Prot.K + SDS)
+ PMSF
PCR
Index-PCR
Sequencing (NGS)

Fig. 2C

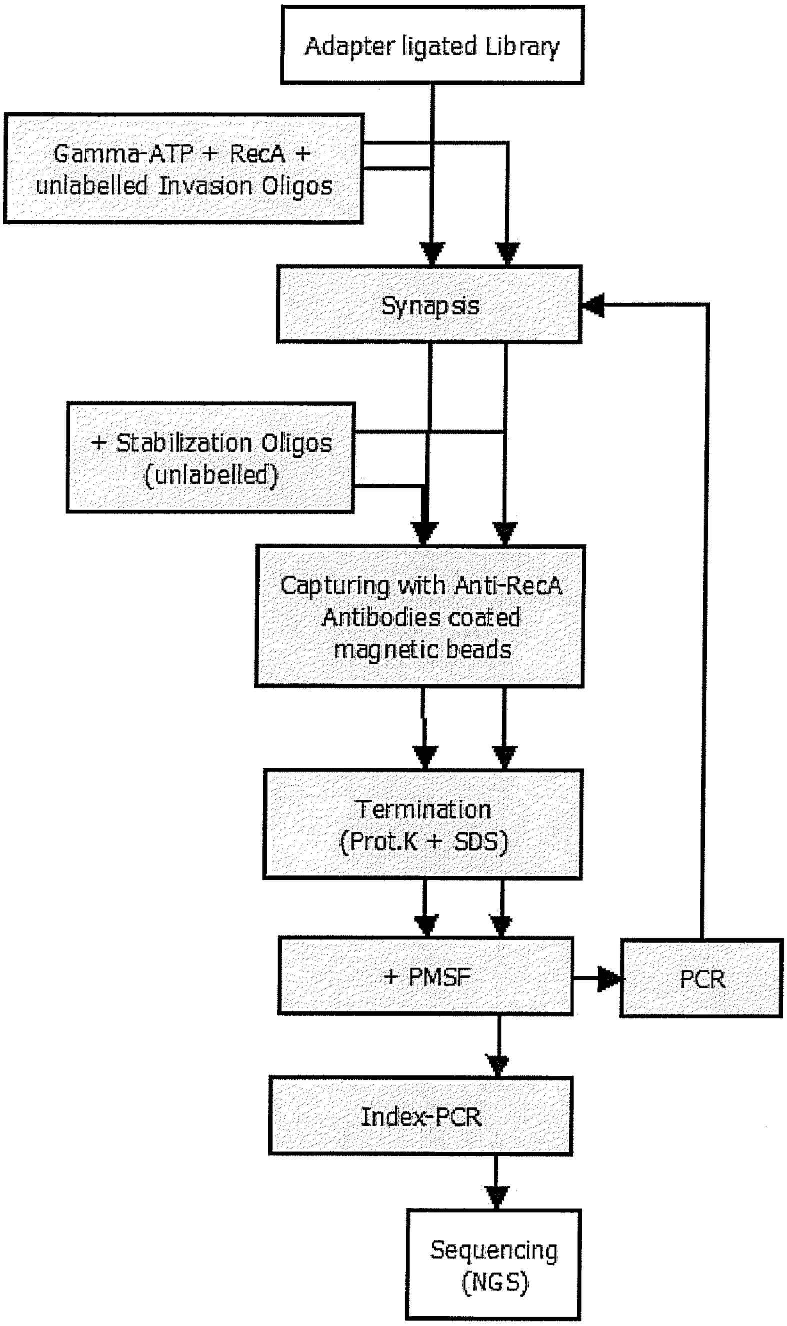
Adapter ligated Library
Gamma-ATP + RecA + unlabelled Invasion Oligos
Synapsis
+ Stabilization Oligos (unlabelled)
Capturing with Anti-RecA Antibodies coated magnetic beads
Termination (Prot.K + SDS)
+ PMSF
PCR
Index-PCR
Sequencing (NGS)

Fig. 2D

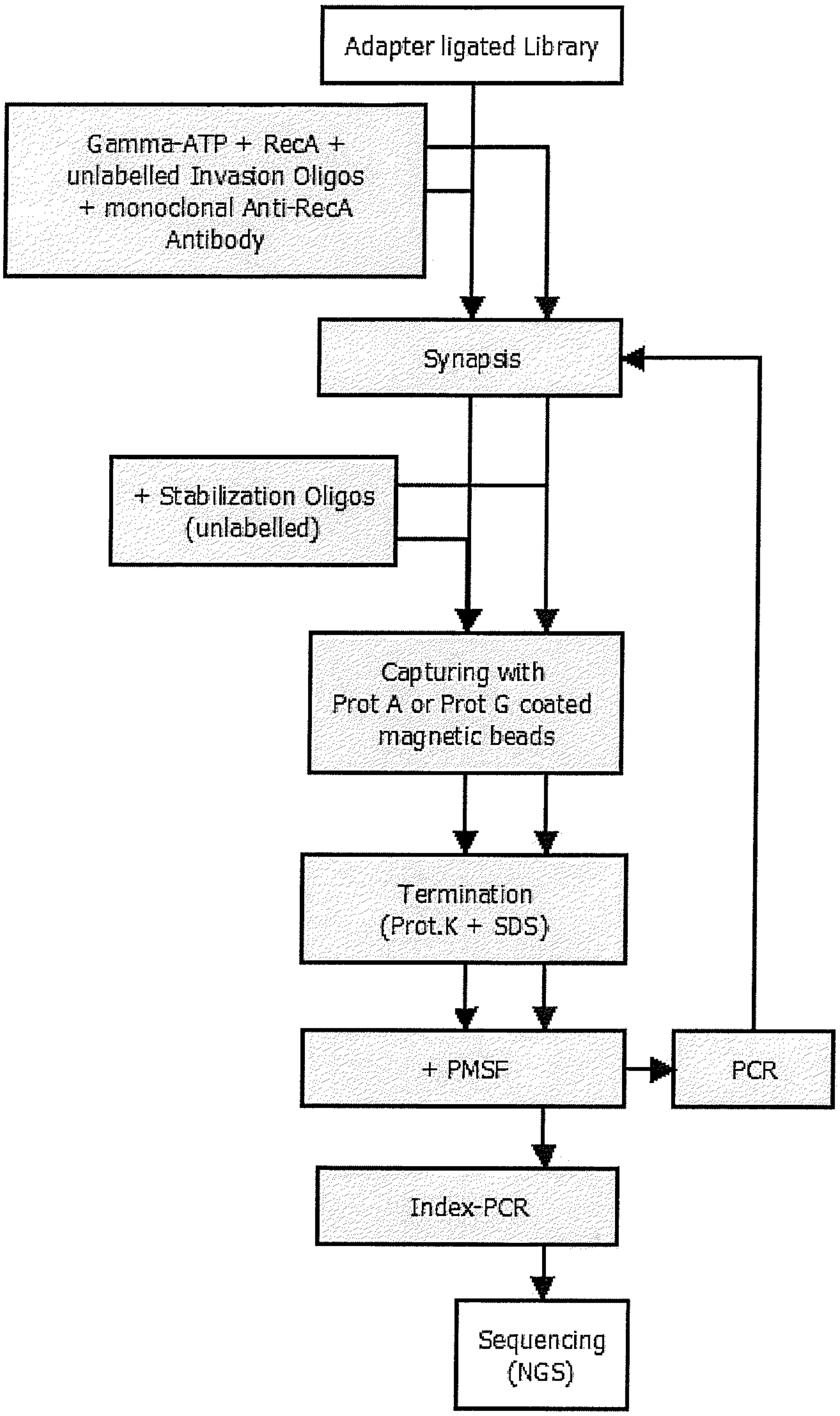
Adapter ligated Library
Gamma-ATP + RecA + unlabelled Invasion Oligos + monoclonal Anti-RecA Antibody
Synapsis
+ Stabilization Oligos (unlabelled)
Capturing with Prot A or Prot G coated magnetic beads
Termination (Prot.K + SDS)
+ PMSF
PCR
Index-PCR
Sequencing (NGS)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100029498 A
- WO 8701730 A
- WO 9808975 A
- US 7741463 B
- US 20090298075 A
- WO 12003374 A
- WO 02079495 A
- US 61678818 B
- EP 12179098 A


### Non-patent literature cited in the description


- **VOELKERDING et al.** *Clinical Chemistry,* 2009, vol. 55 (4), 641-658
- **METZKER.** *Nature Reviews/ Genetics,* January 2010, vol. 11, 31-46
- **MERTES et al.** *Briefings in Functional Genomics,* 2011
- **SHEN et al.** *Proc. Natl. Acad. Sci. U.S.A.,* 2011, vol. 108, 6549-6554
- **ALBERT et al.** *Nature Methods,* 2007, vol. 4, 903-905
- **ZHUMABAYEVA.** *Biotechniques,* 1999, vol. 27, 834-845
- **SENA et al.** *nature genetics,* 1993, vol. 3, 365-372
- **KOWALCZYKOWSKI,S.** *Ann. Rev. Biohpys. Biophysical Chem.,* 1991, vol. 20, 539-575
- **RADDING C., M.** *Biochem. Biophys. Acta,* 1989, vol. 1008, 131-139
- **RADDING C., M.** *J. Biol. Chem.,* 1991, vol. 266, 5355-5358
- **GOLUB, E. et al.** *Nucleic Acids Res.,* 1992, vol. 20, 3121-3125
- **KIRKPATRICK, S. et al.** *Nucleic Acids Res.,* 1992, vol. 20, 4339-4346
- **SHIBATA T. et al.** *Method in Enzymology,* 1983, vol. 100, 197
- **MADIRAJU M. et al.** *Proc. Natl. Acad. Sci. USA,* 1988, vol. 85, 6592
- **KAWASHIMA H. et al.** *Mol. Gen. Genet.,* 1984, vol. 193, 288
- **YONCSAKI T.** *Eur. J. Biochem.,* 1985, vol. 148, 127
- **LOVETT C. M. et al.** *J. Biol. Chem.,* 1985, vol. 260, 3305
- **KMIEC E. B. et al.** *Cell,* 1982, vol. 29, 367
- **ANGOV E. et al.** *J. Bacteriol.,* 1994, vol. 176, 1405
- **KATO R. et al.** *J. Biochem.,* 1993, vol. 114, 926
- **SHINOHARA A. et al.** *Nature Genetics,* 1993, vol. 4, 239
- **SENER et al.** *Belozerkowskii, Biochemistry 1999,* 1993, vol. 38, 10.785-10.792
- **GENOV et al.** *Int. J. Peptide Protein Res.,* 1995, vol. 45, 391-400